# EUROPEAN PATENT APPLICATION

(11) **EP 4 435 088 A1**
(43) Date of publication of application: **25.09.2024**
(21) Application number: 23382263.4
(22) Date of filing: 22.03.2023
(51) Int. Cl.: C12M 3/06, C12M 1/00, C12M 1/12

(54) **CELLULAR SUPPORT FOR CULTURING METHODS**

(71) Applicant: Universidad del País Vasco/Euskal Herriko Unibertsitatea, 48940 Leoia, Bikaia (ES)
(72) Inventor: BASABE DESMONTS, Lourdes, E-48940 LEIOA - VIZCAYA (ES); BENITO LÓPEZ, Fernando, E-48940 LEIOA - VIZCAYA (ES); AZUAJE HUALDE, Enrique, E-48940 LEIOA - VIZCAYA (ES); MARTÍNEZ DE PANCORBO GÓMEZ, María de los Ángeles, E-48940 LEIOA - VIZCAYA (ES); ÁLVAREZ BRAÑA, Yara, E-48940 LEIOA - VIZCAYA (ES); ALONSO CABRERA, Juncal, E-48940 LEIOA - VIZCAYA (ES); LARTITEGUI MENESES, Naiara, E-48940 LEIOA - VIZCAYA (ES); SAEZ CASTAÑO, Janire, E-48940 LEIOA - VIZCAYA (ES)
(74) Representative: ABG Intellectual Property Law, S.L.

(57) **Abstract**

The inventors of the present invention have developed a support that couples the controlled seeding of cells into predetermined patterns with the presentation and/or detection of molecules of interest to/from said cells. Said support is characterized for containing predetermined patterns where cells adhere to and have a uniform layer of gel functionalized with a molecule of interest or an uniform layer of gel and microbeads functionalized with a molecule of interest.

## Description

### TECHNICAL FIELD OF THE INVENTION

The present invention relates to the field of cellular biology and, more in particular, to assays for the study of the interaction of cells with the components of the extracellular matrix as well as for the analysis of compounds which are secreted by cells.

### BACKGROUND OF THE INVENTION

The cell microenvironment, made off the extracellular matrix (ECM), other surrounding cells, and the cell secreted signaling molecules, plays a key role in cell's development, growth and migration and is essential in proper physiological and pathological processes. Two-dimensional cell culture methods, the gold standard technique for studying cells in vitro, lacks the capability to control or imitate physiological cell microenvironments. Substrate stiffness and porosity, biochemical composition of the matrix, solid-phase anchoring of signaling molecules and the three-dimensional cell architectures are some of the parameters that are overlooked in conventional cell culture. This drawback heavily limits the possibility to study and understand cells inner processes in vitro. For such reason, interest has risen in generating methods and technologies that enable the culture of cells within recreated cell microenvironments for the more accurate study of the many cell interactions, including cell-cell, cell-matrix and cell-secreted factors interactions.

Many technologies have arisen that aim to achieve the aforementioned control over cell microenvironment, including the patterning of cells for guiding cell-material and cell-cell interactions; the three-dimensional (3D) printing of biomaterials; or the replication of whole physiological systems on chips (organ-on-a-chip). Among these, 3D bioprinting is one of the leading techniques in tissue and organ engineering. The 3D extracellular framework enables to mimic the structural complexity of the matrices found in physiological conditions with high fidelity. The capability to control the structuration of biomaterials enables both the creation of cell-specific biochemical matrices, and the spatial control in the deposition, adhesion and architecture of one or several cell types. However, most common 3D bioprinting techniques, such as extrusion-based or laser-based 3D bioprinting, does not possess the resolution required to generate features at the micron and submicron scale, which is essential for the accurate localization of the cells within the matrix. Furthermore, these conventional techniques are not designed for the fabrication of multicomponent 3D networks. Finally, while interest have increased in developing novel technologies and platforms that enables both the imitation of 3D complex cell microenvironments, and the monitoring of their behavior, the integration of biosensors for the monitoring of cell secretion inside these novel platforms has not been widely achieved due to several limitations that are presented in conventional methodologies.

Therefore, new devices and methods are required which allow for the accurate recreation of cell microenvironments which allow the study of cell-cell, cell-matrix and cell-secreted factors interactions.

### SUMMARY OF THE INVENTION

The inventors of the present invention have developed a support that enables the multicomponent patterning of 3D gel structures with high spatial resolution, in combination with other 2D and 3D structures including cell-binding molecules and functionalizes microbeads.

As such, a first aspect of the present invention relates to a support suitable for culturing cells characterized in that it shows patterned regions, said patterned regions being defined by:
- a complementary pattern of a uniformly distributed gel attached to the support, wherein the gel comprises at least one first functionalizing molecule , or
- a complementary pattern comprising two or more complementary patterned regions, wherein at least one complementary pattern region is defined by an uniformly distributed gel attached to the support and at least one complementary pattern region is defined by uniformly distributed microbeads attached to the support, wherein the gel comprises at least one first functionalizing molecule and/or the microbeads are modified with at least one second functionalizing molecule,
wherein the patterned regions in the support show increased adhesive capacity to cells with respect to the complementary pattern to which the gel or the gel and microbeads are attached.

Another aspect relates to a method for producing the support according to the invention selected from:
(a) a method comprising the steps of:
   (i) Providing a support;
   (ii) Contacting the support with a stamp, said stamp having protrusions which define the desired pattern in the support, wherein the contact of the support with the stamp forms bottomless channels between the support and the stamp,
   (iii) contacting the support along the bottomless channels with a gel solution and allowing the gel solution to distribute uniformly in those regions in the support which are not protected by the protrusions of the stamp, and
   (iv) polymerizing the gel in order for the gel to adhere to the support as to obtain a uniform attachment onto those regions in the support which are not protected by the protrusions of the stamp obtaining a pattern defined by the gel which is complementary to the desired pattern,
   wherein the gel solution comprises at least one first functionalizing molecule or at least one first functionalizing molecule is added to the gel after the gel is polymerized;
   or
(b) a method comprising the steps of:
   (i) Providing a support;
   (ii) Contacting the support with a stamp, said stamp having protrusions which define the desired pattern in the support, wherein the contact of the support with the stamp forms bottomless channels between the support and the stamp,
   (iii) contacting the support along the bottomless channels with a gel solution and a microbead suspension wherein the microbeads are modified with at least one second functionalizing molecule and allowing the microbeads to adhere to the support as to obtain a uniform deposition in and attachment of the microbeads onto those regions in the support which are not protected by the protrusions of the stamp nor occupied by the gel solution, and
   (iv) and polymerizing the gel in order for the gel to adhere to the support as to obtain a uniform attachment onto those regions in the support which are not protected by the protrusions of the stamp nor occupied by the microbeads, obtaining a pattern defined by the gel and microbeads which is complementary to the desired pattern,

   wherein the gel comprises at least one first functionalizing molecule and/or the microbeads are modified with at least one second functionalizing molecule and
   wherein the gel solution comprises at least one first functionalizing molecule or at least one first functionalizing molecule is added to the gel after the gel is polymerized.

A further aspect of the present invention relates to a method for determining the effect of a molecule on a cell population which comprises the steps of:
(i) Providing a support according to the invention wherein said support contains the cell population wherein the cells of the population are attached to the support and wherein the molecule, the effect of which on the cell population is to be determined is the at least one first functionalizing molecule and/or the at least one second functionalizing molecule;
(ii) Maintaining the support under adequate conditions to allow the at least one first functionalizing molecule and/or the at least one second functionalizing molecule to exert its effect on the cells of the cell population; and
(iii) Detecting the response of the cells within the cell population to said first functionalizing molecule and/or the at least one second functionalizing molecule,
wherein the detection of a response of the cells to the at least one first functionalizing molecule and/or the at least one second functionalizing molecule is indicative that the at least one first functionalizing molecule and/or the at least one second functionalizing molecule exerts an effect on the cells of the cell population.

Another aspect of the present invention relates to a method for determining if a cell population secretes to the medium a substance of interest which comprises the steps of:
(i) Providing a support according to the invention wherein said support contains a cell population wherein the cells of the population are attached to the support and wherein the at least one first functionalizing molecule and/or the at least one second functionalizing molecule comprises a molecule which is capable of interacting with the substance of interest, said interaction resulting in a detectable signal;
(ii) Culturing the cells in the adequate conditions to allow binding of the substances of interest secreted to the medium to the at least one first functionalizing molecule and/or the at least one second functionalizing molecule; and
(iii) Detecting the binding of the substance of interest to the at least one first functionalizing molecule and/or the at least one second functionalizing molecule by detecting the signal resulting from said interaction.

A further aspect of the present invention relates to a method for determining the effect of an effector molecule on a cell population and for determining if the cell population secretes to the medium a substance of interest which comprises the steps of:
(i) Providing a support according to the invention wherein said support contains the cell population, wherein the cells of the population are attached to the support and wherein:
   - the gel comprises a first type of at least one first functionalizing molecule which is the effector molecule and a second type of at least one first functionalizing molecule which is capable of interacting with the substance of interest;
      the gel comprises a first type of at least one first functionalizing molecule which is the effector molecule and the microbeads comprise a second type of at least one second functionalizing molecule which is capable of interacting with the substance of interest;
   - the microbeads comprise a first type of at least one second functionalizing molecule which is the effector molecule and the gel comprises a second type of at least one first functionalizing molecule which is capable of interacting with the substance of interest; or
   - the first and second type of microbeads contain, respectively, a first type of second functionalizing molecule which is the effector molecule and a second type of second functionalizing molecule which is capable of interacting with the substance of interest,
      and wherein the interaction of the first functionalizing molecule or of the second functionalizing molecule with the substance of interest results in a detectable signal;
(ii) Culturing the cells under conditions adequate to allow the effector molecule to exert its effect on the cells of the cell population and the binding of the substances of interest secreted to the medium to the detector molecule; and
(iii) Detecting the response of the cells within the cell population to said effector molecule and detecting the binding of the substance of interest to the detector molecule by detecting the signal resulting from said interaction.

### BRIEF DESCRIPTION OF THE FIGURES

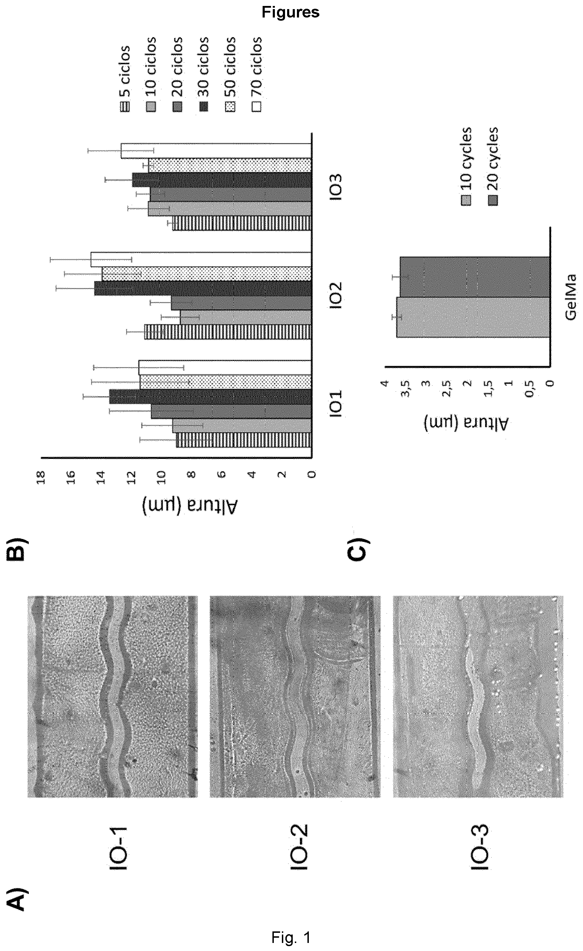

**Figure 1****.** Patterning and polymerization of gels through vacuum lithography. A) Brightfield photographs of IO-1, IO-2 and IO-3 microstructures (10 cycles UV light). B) Graphic of the height of the ionogel microstructures generated by 5, 10, 20, 30, 50 and 70 cycles of UV light, measured with a contact profilometer (n=3 per condition). C) Height of GelMa microstructures generated by 10 and 20 cycles of UV light and measured with a contact profilometer (n=3 per condition).

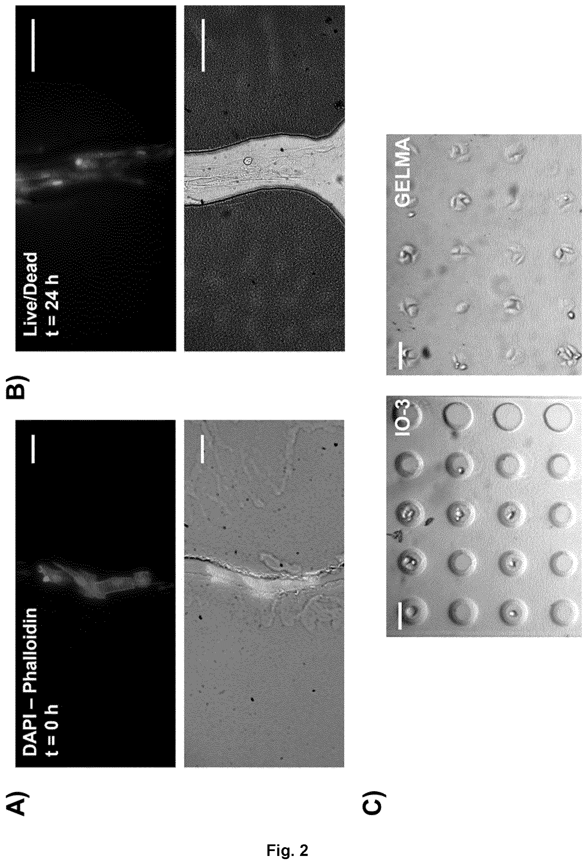

**Figure 2****.** Combined patterns of gels and cells obtained through PnVlitho) A) Brightfield (bottom) and Fluorescence (top) images of the co-patterns of IO-3 microstructures and hHF-MSCs. Cell nucleus and cytoskeleton were stained with DAPI and phalloidin, respectively. Scale bars equals 100 µm. B) Brightfield (bottom) and Fluorescence (top) images of the Live/Dead viability test assays performed in the samples after being cultured for 24 h. Scale bars equals 100 µm. C) Brightfield images of the adhesion of hHF-MSCs to the 100 µm dots surrounded by either IO-3 o GelMa microstructures. Scale bar equals 100 µm.

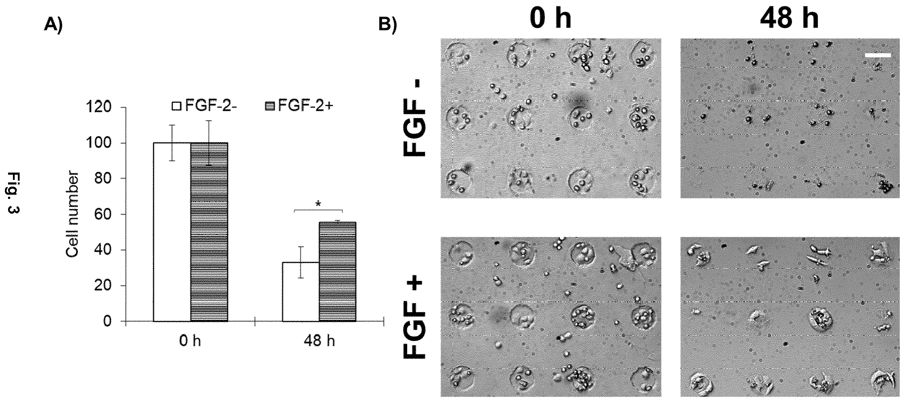

**Figure 3****.** Presentation of FGF-2 loaded GelMa on patterned HeLa cells. A) Graphic of the normalize number of HeLa cells (%) after patterning (0 h) and 48 h after in GelMa patterns (FGF -) and GelMa + FGF-2 patterns (FGF +). * means statistical differences of p ≤ 0.05. B) Brightfield microscopy images of the combine patterns of GelMa and HeLa after adhesion and after 48 h. Scale bar equals 100 µm.

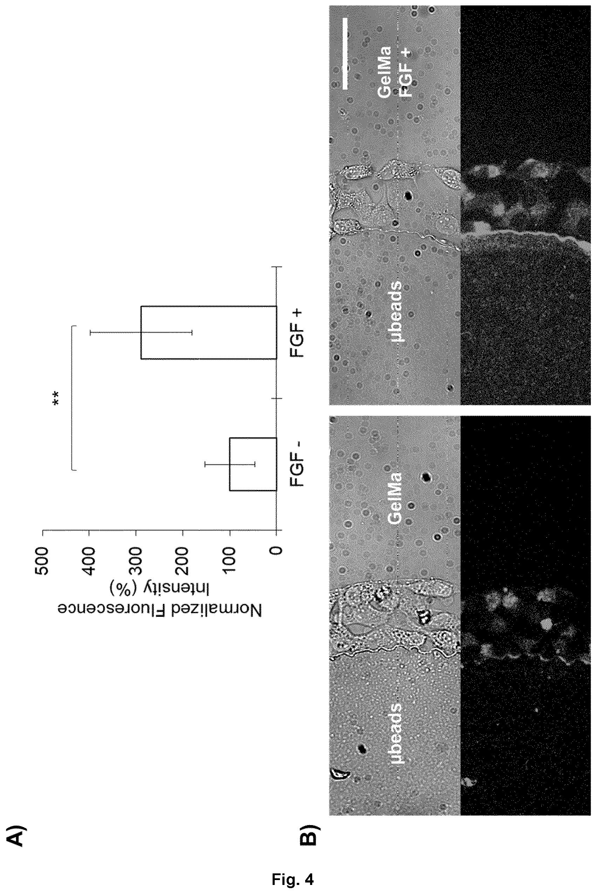

**Figure 4****.** Effect of FGF-2 loaded GelMa on VEGF secretion by HeLa cells. A) Graphic of the normalized Fluorescence intensity of VEGF secretion cells (%) in GELMA patterns (FGF -) and GelMa + FGF-2 patterns (FGF +). B) Brightfield and Fluorescence microscopy images of the HeLa patterns (middle of image) between microbeads patterns (left of the images) and the GELMA patterns (right of the image). Scale bar equals 100 µm.

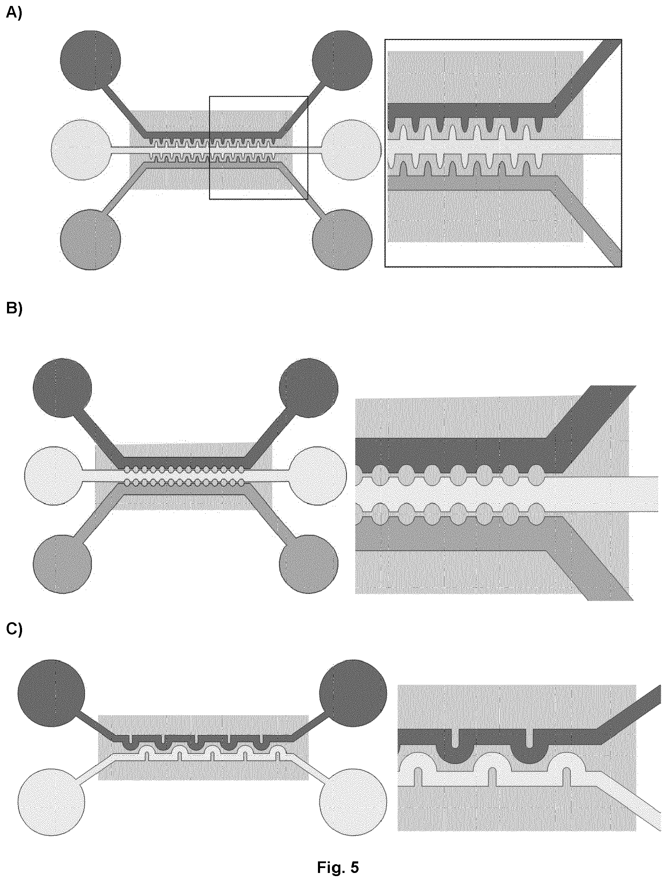

**Figure 5****.** Examples of supports with different number of chambers and different pattern regions. **A.** Support with 3 chambers with inlets and outlets (left panel) forming a pattern region of protuberances (right panel). **B.** Support with 3 chambers with inlets and outlets (left panel) forming a pattern region of circles (right panel). **C.** Support with 2 chambers with inlets and outlets (left panel) forming a pattern region of invaginations (right panel).

### DETAILED DESCRIPTION OF THE INVENTION

The inventors of the present invention have developed a support that couples the controlled seeding of cells into predetermined patterns with the presentation and/or detection of molecules of interest to/from said cells. Said support presents several advantages related to previous ones such as precise control of the position and number of cells present in the predetermined patterns as well as presentation and or detection of molecules of interest due to the uniform covering of the support surface with a gel functionalized with at least one molecule of interest. Furthermore, the uniform covering of the support surface with a gel which has 3D properties allows for the culturing of cells in the support in 3D as well, while the possibility of having the support covered partly with gel and partly with microbeads allows the analysis of response parameters of the cells to different molecules of interest as well as different culture environments.

The support of the present invention allow the creation of two- and three-dimensional patterns of different chemical compositions quickly with high spatial resolution, including gel patterns to stimulate the cells, protein patterns for cell adhesion and microbead patterns for cell secretion biosensing. The present support can combine protein, microbeads and gel patterns for the maintenance of cell arrays with controlled chemical cues and the monitoring of cell events down to a cell secretion level.

### Support

Therefore a first aspect of the present invention relates to a support suitable for culturing cell, from here onwards, "support of the invention", characterized in that it shows patterned regions, said patterned regions being defined by:
- a complementary pattern of a uniformly distributed gel attached to the support, wherein the gel comprises at least one first functionalizing molecule, or
- a complementary pattern comprising two or more complementary patterned regions, wherein at least one complementary pattern region is defined by an uniformly distributed gel attached to the support and at least one complementary pattern region is defined by uniformly distributed microbeads attached to the support, wherein the gel comprises at least one first functionalizing molecule and/or the microbeads are modified with at least one second functionalizing molecule,
wherein the patterned regions in the support show increased adhesive capacity to cells with respect to the complementary pattern to which the gel or the gel and microbeads are attached.

The term "support" as used herein refers to a structure acting as a base which is able to support, sustain or maintain a cell or cells. The support as used herein can be made of any appropriate material such thermoplastics, thermosets, elastomers such as epoxy, phenolic, PDMS, glass, silicones, nylon, polyethylene, polystyrene, cyclic olefin copolymers or any other suitable material. In a preferred embodiment of the support of the invention, the support is made from a material selected from a group consisting of: glass, borosilicate glass, quartz glass, polystyrene, polymethylmethacrylate (PMMA), polycarbonate, polyethylene and cyclic olefin copolymers. In a more preferred embodiment the support is made of glass.

The expression "suitable for culturing cells" as used herein refers to any material in which cellular material is stored or maintained in a controlled environment so as to produce the conditions required for viability, i.e., the processes of cell culture. In a preferred embodiment the support is composed of a material which is hydrophilic, cytophilic and/or biocompatible. The term "hydrophilic" as used herein refers to a material which is attracted to or has an affinity to water. The term "cytophilic" as used herein refers to a material which is attracted to or has an affinity to cells. The term biocompatible as used herein refers to a material which has the ability to contact with a living system, such as cells, without producing adverse effects.

The term "cell" as used herein is to be understood as interchangeable with the term "cellular material" and shall refer to a cell, group of cells, tissue, or organoid which is the subject of the invention described herein.

The term "patterned regions" as used herein refers to any type of free or geometric form which can be imprinted/established into the support material which determines the bounds of two regions which contain or do not contain gel or gel and microbeads. The term "patterned regions" does not imply that the form present in the support of the invention contains a motif which is repeated, as any free form or geometric form with or without a repeated motif is allowed. The patterned regions can be defined, without limitation, as circles, proteburances, invaginations, triangles, squares, rectangles, ovals, etc. In a particular embodiment the pattern regions are defined as depicted in Figure 5. The patterned regions of the support of the invention can be defined by a complementary pattern of a uniformly distributed gel attached to the support, wherein the gel comprises at least one first functionalizing molecule.

The term "complementary pattern" as used herein refers to a pattern whose form is the exact opposite to the pattern of the patterned regions, wherein both patterns have a relationship which follows the lock-and-ley principle.

The complementary pattern can be defined either by a uniformly distributed gel or by two or more patterned regions, wherein at least one patterned region is defined by a uniformly distributed gel and at least one region is defined by a uniformly distributed microbeads.

The term "gel" as used herein encompasses its ordinary meaning in the art. The term "gel" as used herein can refer to a composition comprising a viscous polymer having a fluidity at room temperature between that of a liquid and a solid. As such, the term "gel" refers to a solid or semisolid colloid system formed of a solid continuous phase and a liquid phase (either discontinuous or continuous or mixed), which, in some embodiments, can be identified by its outward gelatinous appearance, and/or exhibits properties of a solid such as plasticity, elasticity, or rigidity. The term "gel" as used in the present context refers to both hydrogel, as well as ionogel. In a particular embodiment of the support of the invention the gel is a hydrogel or an ionogel.

The term "hydrogel" in the present context refers to a three-dimensional, hydrophilic or amphiphilic polymeric network that holds together and comprises water as its major component (and/or is capable of taking up large quantities of water). As used herein, the networks are composed of polymers (such as polypeptides) and are insoluble due to the presence of covalent chemical or physical (ionic, hydrophobic interactions, entanglements) crosslinks. The crosslinks provide the network structure and physical integrity that is useful when used as a scaffold (such as bio-scaffold). Hydrogels exhibit a thermodynamic compatibility with water that allows them to swell in aqueous media. In a particular embodiment of the support of the invention the gel is a hydrogel comprising a polymer selected from a group consisting of: hyaluronans, agar, heparin, sulfate, cellulose, alginates (including alginate sulfate), collagen, dextrans (including dextran sulfate), pectin, polylysine, gelatins (including gelatin type A), (meth)acrylate-oligolactide-PEO-oligolactide- (meth)acrylate, PEO-PPO-PEO copolymers (Pluronics), poly(phosphazene), poly(methacrylates), poly(N-vinylpyrrolidone), PL(G)A-PEO-PL(G)A copolymers, poly(ethylene imine), polyethylene glycol (PEG)-thiol, PEG-acrylate, acrylamide, N,N'- bis(acryloyl)cystamine, PEG, polypropylene oxide (PPO), polyacrylic acid, poly(hydroxyethyl methacrylate) (PHEMA), poly(methyl methacrylate) (PMMA), poly(N- isopropyl acrylamide) (PNIPAAm) and its derivates such as CS-g-PNIPAM and AALG-g-PNIPAM, poly(lactic acid) (PLA), poly(lactic-co-glycolic acid) (PLGA), polycaprolactone (PCL), poly(vinylsulfonic acid) (PVSA), poly(L-aspartic acid), diacrylate, diallylamine, triallylamine, divinyl sulfone, diethyleneglycol diallyl ether, ethyleneglycol diacrylate, polymethyleneglycol diacrylate, polyethyleneglycol diacrylate, trimethylopropoane trimethacrylate, ethoxylated trimethylol triacrylate, or ethoxylated pentaerythritol tetracrylate, Matrigel^{©}, poly(3,4-ethylenedioxythiophene), poly(3,4-ethylenedioxythiophene), alginic acid, agarose and its synthetically-modified derivates such as carboxylated agarose, fibrin, chitosan and its derivates such as hydroxybutyl chitosan, fibrinogen, fibronectin, laminin, elastin, silk, poly(3,4-ethylenedioxythiophene) _(PEDOT), poly(3,4-ethylenedioxythiophene) polystyrene sulfonate (PEDTOR:PSS), acrylate monomers and oligomers, polyethylene, polyvynil alcohol, poly-2-hydroxyethyl methacrylate, polypropylene, polydimethylsiloxane (PDMS), polyethylene terephthalate, polytetrafluoroethylene, polyglycolic acid (PGA), agarose and its synthetically-modified derivates such as carboxylated agarose, carrageenans, gellan gum, xanthan gum, lactic acid, aminated guar gum, cyclodextrine, laminarin and combinations thereof. In a particular embodiment of the support of the invention the gel is a hydrogel comprising a polymer selected from a group consisting of: alginic acid, agarose, hyaluronic acid, polyethylene glycol, gelatin, collagen, fibrin, chitosan, fibrin, fibrinogen, fibronectin, laminin, elastin, and combinations thereof. Matrigel as used herein refers to solubilized basement membrane matrix secreted by Engelbreth-Holm-Swarm (EHS) mouse sarcoma cells. In a more particular embodiment the hydrogel polymer is gelatin, preferably gelatin methacrylate.

The term "gelatin" as used herein refers to protein substances derived from collagen. In the context of this description, "gelatin" also refers to equivalent substances such as synthetic analogues of gelatin (e.g., gelatin methacrylate (GeIMA)). Generally, gelatin may be classified as alkaline gelatin, acidic gelatin, or enzymatic gelatin. Alkaline gelatin may be obtained from the treatment of collagen with a base such as sodium hydroxide or calcium hydroxide. Acidic gelatin may be obtained from the treatment of collagen with an acid such as hydrochloric acid. Enzymatic gelatin may be obtained from the treatment of collagen with an enzyme such as hydrolase. In a preferred embodiment the hydrogel comprises gelatin methacrylate and lithium phenyl-2,4,6-trimethylbenzoylphosphinate in a HEPES buffer solution.

The term "ionic liquid" refers to the association of cation and anion in the liquid state at temperatures close to room temperature. The term "ionogel" as used herein refers to continuous solid skeleton containing an ionic liquid. In a particular embodiment of the support of the invention the gel is an ionogel comprising an ionic liquid selected from the group consisting of: 1-methyl-3-butylimidazole tetrafluoroborate, 1-methyl-3-butylimidazole hexafluorophosphate, and 1-ethyl-3-methylimidazole ester Acid salt, 1-methyl-butylimidazole hydrogen sulfate, 1-ethyl-3-methylimidazole bistrifluoromethanesulfonimide salt, 1-ethyl-3-methylimidazole dicyanamide 1-ethyl-3-methylimidazolium ethyl sulphate, trihexyltetradecylphosphonium dicyanamide, choline acetate, 1-butyl-2,3-dimethylimidazolium, 1-butyl-3-methylimidazolium, 1-butyl-1-methylpyrrolidinium, 1-butyl-1-methylpiperidinium, and methyltrioctylammonium.

The ionogel may further comprise other components such as a polymer and a crosslinker. In a particular embodiment the gel is an ionogel, wherein the ionogel comprises an ionic liquid, a polymer and at least one crosslinker.

In a particular embodiment of the support of the invention the gel is an ionogel comprising a polymer selected from a group consisting of: 2-hydroxyethyl acrylate, 2-methoxyethyl acrylate, acrylamide, N-isopropylacrylamide, N, N-dimethylacrylamide, N-Methylol acrylamide.

The term "crosslinker" refers to a molecule that can form a three-dimensional network when reacted with the appropriate base monomers. In a particular embodiment of the support of the invention the gel is an ionogel comprising a crosslinkers selected from a group consisting of: N,N'-methylene-bis(acrylamide), citric acid, vanillin, gallic acid, ferulic acid, genipin, glutaraldehyde, methylene bis-acrylamide, 1,2,3,4-butanetetracarboxylic dianhydride, N-(3-Dimethylaminopropyl)-N'-ethyl carbodiimide hydrochloride, 2-chloro-1-methylpyrinium iodide, 2,2-dimethoxy-2-phenylacetophenone and any combination thereof. The term "photoinitiator" or "photocatalist" refers to a substance which is activated by light irradiation to generate a free radical, thereby causing a polymerization reaction of a polymer.

In a particular embodiment of the support of the invention the gel is an ionogel wherein the ionogel comprises an ionic liquid, a polymer and at least one crosslinker. In another particular embodiment the ionogel comprises an ionic liquid, a polymer and two crosslinkers.

In another particular embodiment of the support of the invention the gel is an ionogel comprising an ionic liquid, a polymer, one crosslinker and one photoinitiator, wherein the ionic liquid is 1-ethyl-3-methylimidazolium ethyl sulphate, the polymer is N-isopropylacrylamide, the crosslinker is N,N'-methylene-bis(acrylamide) and the photoinitiator is 2,2-dimethoxy-2-phenylacetophenone, at a ratio of ionic liquid (in mL) to polymer (in grams) of about 0.4 to about 0.5, preferably 0.45, a ratio of ionic liquid (in mL) to crosslinker or photoinitiator (in grams) of about 0.025 to about 0.035, preferably about 0.03.

In another particular embodiment of the support of the invention the gel is an ionogel comprising an ionic liquid, a polymer, one crosslinker and one photoinitiator, wherein the ionic liquid is trihexyltetradecylphosphonium dicyanamide, the polymer is N-isopropylacrylamide and the crosslinker is N,N'-methylene-bis(acrylamide) and the photoinitiator is 2,2-dimethoxy-2-phenylacetophenone, at a ratio of ionic liquid (in grams) to polymer (in grams) of about 0.25 to about 0.35, preferably 0.30, a ratio of ionic liquid (in grams) to crosslinker or photoinitiator (in grams) of about 0.015 to about 0.025, preferably about 0.02.

In another particular embodiment of the support of the invention the gel is an ionogel comprising an ionic liquid, a polymer, one crosslinker and one photoinitiator, wherein the ionic liquid is choline acetate, the polymer is N-isopropylacrylamide and the crosslinker is N,N'-methylene-bis(acrylamide) and the photoinitiator is 2,2-dimethoxy-2-phenylacetophenone, at a ratio of ionic liquid (in mL) to polymer (in grams) of about 0.4 to about 0.5, preferably 0.45, a ratio of ionic liquid (in mL) to the crosslinker or the photoinitiatior (in grams) of about 0.025 to about 0.035, preferably about 0.03.

The expression "uniformly distributed gel attached to the support" as used herein refers to the homogenous distribution of the gel within the complementary pattern in the support surface. The gel can be considered as being uniformly distributed over the complementary pattern, if the complementary pattern is divided into areas of equal surface, the volume of gel in each area is substantially constant among the different areas. Thus, in some embodiments, the volume of gel within two different areas varies by less than 10%, less than 9%, less than 8%, less than 7%, less than 6%, less than 5%, less than 4%, less than 3%, less than 2%, less than 1%, less than 0,5%, less than 0,1%, less than 0,05%, less than 0,01% or even less. As the person skilled in the field will be aware, in order to obtain a uniform distribution of the gel in the support of the invention, the volume of gel must be adjusted as to completely fill with the gel solution the area of the bottomless channels between the support and the stamp solution (see below).

The expert will be aware that in order to distribute the gel over the complementary pattern the gel is first produced as a solution which is liquid or liquefied and introduced into the complementary pattern and will then be let to adhere onto the support and the temperature of the gel will be altered in order for it to jellify. In a particular embodiment of the support of the invention the gel solution is introduced into the complementary pattern at a temperature wherein the gel is or behaves as a liquid. In a particular embodiment of the support of the invention the gel solution is introduced into the complementary pattern at a temperature of between about 40°C and about 70°C, preferably about 41°C, about 42°C, about 43°C, about 44°C, about 45°C, about 46°C, about 47°C, about 48°C, about 49°C, about 50°C, about 51°C, about 52°C, about 53°C, about 54°C, about 55°C, about 56°C, about 57°C, about 58°C, about 59°C, about 60°C, about 61°C, about 62°C, about 63°C, about 64°C, about 65°C, about 66°C, about 67°C, about 68°C, about 69°C.

In another particular embodiment of the support of the invention the gel solution is introduced into the complementary pattern at a temperature of between about 0°C and about 30°C, preferably about 1°C, about 2°C, about 3°C, about 4°C, about 5°C, about 6°C, about 7°C, about 8°C, about 9°C, about 10°C, about 11°C, about 12°C, about 13°C, about 14°C, about 15°C, about 16°C, about 17°C, about 18°C, about 19°C, about 20°C, about 21°C, about 22°C, about 23°C, about 24°C, about 25°C, about 26°C, about 27°C, about 28°C, about 29°C.

In a particular embodiment of the support of the invention the gel is allowed to form by a transition from the sol form to the gel form.

The term "polymerized" as used herein refers to the process of transforming the gel solution into a gel which a consistence more close to a solid then the original gel solution. In a particular embodiment the gel is polymerized by placing the gel solution in conditions which favor polymerization.

The expert in the field will be aware what type of conditions lead the gel solution to polymerize and how to modify the conditions at which the gel solution is exposed to obtain gel polymerization. Examples of modification that may lead to the polymerization of a gel solution into a gel are described in the literature (Akhtar et al, 2016, Saudi Pharmaceutical Journal, 24, 554-559, Bashir et al., 2020, Polymers, 12(11): 2702), describing, amongst others, temperature shifts by either increasing temperature or decreasing temperature, use of photocatalists driven with light in the visible range or the UV/infrared range, enzyme driven polymerization, crystallization, chemical crosslinking, etc.

In a particular embodiment the gel is polymerized from a gel solution through UV irradiation.

The term "UV irradiation" as used herein refers to the process of illuminating or bathing an object with short-wavelength ultraviolet light in order to promote the crosslinking of the polymers present in the gel solution leading it to solidify and harden. In a particular embodiment of the support of the invention the gel is solidified with UV irradiation, wherein the UV irradiation is carried out with a wavelength of between 300 nm and 450 nm, preferably
365 nm and 405 nm. In a particular embodiment of the support of the invention the gel is solidified with UV irradiation with a wavelength of between 300 nm and 450 nm, preferably between 365 nm and 405 nm, wherein the irradiation is carried out in cycles, each cycle consisting of 5 seconds (s) of irradiation (ON) followed by 30 seconds without irradiation (OFF), for between 5 to 70 cycles, preferably for 10, 15, 20, 25, 30, 35, 40, 45, 50, 55, 60, 65 cycles.

In another particular embodiment the UV irradiation is carried out continuously with a wavelength of between 300 nm and 450 nm, preferably 365 nm and 405 nm, for about 1 min, about 1 min, about 2 min, about 3 min, about 4 min, about 5 min, about 6 min, about 7 min, about 8 min, about 9 min, about 10 min, about 11 min, about 12 min, about 13 min, about 14 min, about 15 min, about 16 min, about 17 min, about 18 min, about 19 min, about 20 min.

Once a gel is solidified, as is of general knowledge to the expert the gel will gain a shape or form which depends on the surroundings of the gel. In the present case, if the gel is surrounded by a stamp which has bottomless channels (see below), the gel will solidify retaining, at least in part, the shape of said bottomless channels wherein the gel solution was introduced. Therefore, the gel will not only have the width and length of said bottomless channels as well as a height which will depend on the process of solidification. In a particular embodiment of the support of the invention the complementary pattern of a uniformly distributed gel attached to the support has a height of between or the complementary pattern region of a uniformly distributed gel attached to the support about 1 µm and about 200 µm, between about 1 to 200 µm, between about 10 to 190 µm, between about 20 to 180 µm, between about 30 to 170 µm, between about 40 to 160 µm, between about 50 to 150 µm, between about 60 to 140 µm, between about 70 to 130 µm, between about 80 to 120 µm, between about 90 to 110 µm, between about 95 to 100 µm, preferably of about 4 µm, about 5 µm, about 6 µm, about 7 µm, about 8 µm, about 9 µm, about 10 µm, about 11 µm, about 12 µm, about 13 µm, about 14 µm, about 15 µm, about 16 µm, about 17 µm, about 18 µm, about 19 µm.

In another particular embodiment the gel is polymerized by increasing the temperature to between about 35°C to about 39°C, preferably about 36°C, about 37°C, about 38°C.

In a particular embodiment of the support of the invention the gel comprises "at least one first functionalizing molecule". Said expression in the context of the present invention refers to the fact that the gel comprises a functional group or molecule wherein said functional group or molecule allows the detection and/or interaction of a specific molecule of interest in the medium or in the cell surface of cells that may be present in the support. In another particular embodiment of the support of the invention the gel comprises at least two different types of first functionalizing molecules.

As used herein, the term "functionalizing molecule" refers to any molecule which can cause an effect on a cell (hereinafter known as "effector molecule") or which can specifically bind to a molecule of interest (hereinafter known as "detecting molecule"). It will be understood that the chemical nature of the functionalizing molecule is not particularly limiting as long as the molecule can be attached to the microbeads and thus results in the functionalization of the microbead. In some embodiments, the functionalizing molecule is a small organic molecule, a peptide, a polypeptide, a nucleic acid, a carbohydrate or a lipid.

In a preferred embodiment, the first functionalizing molecule is a high molecular weight compound.

As used herein, "high molecular weight compound" refers to any chemical entity or molecule, such as nucleic acids, peptides, proteins, natural and synthetic polymers, drugs, having a molecular weight greater than 1 kDa, preferably greater than 5 kDa, more preferably greater than 10 kDa and even more preferably greater than 100 kDa.

In one embodiment, the first functionalizing molecule is a poplypeptide.

The term "polypeptide", as used herein, refers to a polymer of amino acid residues. The term also apply to amino acid polymers in which one or more amino acid residue is an artificial chemical mimetic of a corresponding naturally occurring amino acid, as well as to naturally occurring amino acid polymers and non-naturally occurring amino acid polymer.

In one embodiment, the first functionalizing molecule is a polynucleotide.

The terms "nucleic acid" and "polynucleotide", as used herein interchangeably, refer to a polymer composed of nucleotide units (ribonucleotides, deoxyribonucleotides, related naturally occurring structural variants and synthetic non-naturally occurring analogs thereof or combinations thereof) linked via phosphodiester bonds, related naturally occurring structural variants and synthetic non-naturally occurring analogs thereof.

In one embodiment, the first functionalizing molecule is a lipid.

The term "lipid" is used to indicate an organic compound that includes an ester of fatty acid or a derivative thereof and is characterized by being insoluble in water, but soluble in many organic solvents. Suitable lipids for use as functionalizing molecules include, without limitation, fatty acyls (FA), such as fatty acids, fatty acyl carnitines and fatty acyl Coenzyme As, glycerolipids (GL), such as monoacylglycerols, cardiolipins, diacylglycerols and triacylglicerols, glycerophospholipids (GPL) such as phosphatidylcholines, phosphatidylethanoloamines, and bis(monoacylglycerolphospahte (BMP), sphingolipids (SPL) such as ceramies, sphonglmyelins and cholesterylester, and sterol lipids (ST).

In another embodiment, the first functionalizing molecule is a small organic molecule. In general, a "small molecule" refers to a substantially non-peptidic, non-oligomeric organic compound either prepared in the laboratory or found in nature. Small molecules, as used herein, can refer to compounds that are "natural product- like," however, the term "small molecule" is not limited to "natural product-like" compounds. Rather, a small molecule is typically characterized in that it contains several carbon-carbon bonds, and has a molecular weight of less than 1500 g/mol, less than 1250 g/mol, less than 1000 g/mol, less than 750 g/mol, less than 500 g/mol, or less than 250 g/mol, although this characterization is not intended to be limiting for the purposes of the present invention. In certain other embodiments, natural-product-like small molecules are utilized.

The functionalization of the gel with a molecule can be accomplished by several methods known to the expert in the field, namely, without limitation, by covalent bonding of the molecule with the gel polymer and/or crosslinker, by embedding the gel with the molecule, by encapsulating the gel with the molecule or by crosslinking the molecule with the gel through chemical reactions such as thiol-maleimide reactions, alkyne-azide reactions, etc, such as described in Han et al., (Gels 2022, 8, 577).

In another particular embodiment of the support of the invention the gel is polymerized in the presence of the at least one first functionalizing molecule. In another particular embodiment of the support of the invention the gel is polymerized in the presence of the at least two first functionalizing molecules. In another particular embodiment of the support of the invention the at least one first functionalizing molecule is added to the gel after the gel polymerization. In yet another particular embodiment of the support of the invention the gel comprises at least two first functionalizing molecules, wherein at least one first functionalizing molecule is present when the gel is polymerized and at least another first functionalizing molecule is added to the gel after the gel polymerization.

The addition of a first functionalizing molecule of interest to the polymerized gel is a process well known to the skilled person in the art. The most common method used is the soaking of the gel in the functionalizing molecules, allowing said molecule to embed the gel pores. Once the gel is removed from the solution, functionalizing molecules will be trapped in the gel where they will be able to interact with other molecules in the medium or the cells surface.

The gel of the support of the invention may further comprise other components which are embedded or encapsulated into the gel, such as microbeads, nanobeads, nanoparticles, etc. In a particular embodiment of the support of the invention the gel further comprises at least one component which is embedded or encapsulated in the gel. In a particular embodiment of the support of the invention the gel further comprises at least two components which is embedded or encapsulated in the gel, wherein at least two components are different between each other. In another particular embodiment of the support of the invention the gel further comprises a component which is embedded or encapsulated in the gel, wherein the component is selected from beads or nanoparticles.

In the context of the present invention the term "beads" refers to a substantially spherical or ellipsoid particle made of a porous biocompatible and/or biodegradable polymeric material, wherein the size of the particle is between 1 µm and 2,000 µm.

The term "nanoparticle" as used herein refers to particles with at least one dimension less than 1 microns. In preferred embodiments, such as for intravenous administration, the nanoparticle is less than 0.1 micron. Nanoparticles do not have to refer to spherical particles, but they can refer to any shape of particles, such as rhomboids, toroid, needle-like, etc.

In a preferred embodiment of the support of the invention the patterned regions are defined by a complementary pattern of an uniformly distributed gel attached to the support and wherein the complementary pattern of the uniformly distributed gel is created by allowing the gel to adhere and solidify onto the support when the support is in contact with a stamp, said stamp having protrusions which define the pattern regions within the support, thereby preventing the gel from attaching to those areas of the support which are in contact with the stamp protrusions. In another particular embodiment of the support of the invention the patterned regions are defined by a complementary pattern comprising two or more complementary pattern regions, wherein at least one complementary pattern region is defined by an uniformly distributed gel attached to the support and at least one complementary patterned region is define by uniformly distributed microbeads attached to the support, wherein the complementary pattern regions are created by allowing the gel and microbeads to adhere and solidify onto the support when the support is in contact with a stamp, said stamp having protrusions which define the patterned regions within the support, thereby preventing the gel and microbeads from attaching to those areas of the support which are in contact with the stamp protrusions.

The terms "stamp" and "slab" are used interchangeably and in the present context refer to elastomeric stamps or slabs which are easily molded or formed into desired patterns or forms and used to allow the formation of the patterns of interest in the surface of the support. Materials suitable to be used as stamps are, without limitation, silicone rubbers (e.g., polydimethylsiloxane (PDMS)), polyurethane rubber, styrene butadiene rubber, and acrylonitrile butadiene rubber, natural rubbers (e.g., poly-cis-isoprene), thermoplastic elastomers (e.g., thermoplastic polyurethane, thermoplastic copolyester, thermoplastic polyamide), epoxies (e.g., SU-8), polyimides, polyurethanes, polyamides, polyesters (e.g., poly(lactic-co-glycolic acid) (PLGA), polylactic acid (PLA), polyglycolic acid (PGA), polycaprolactone (PCL), poly(glycerol sebacate) (PGS)), polysaccharides (e.g., chitosan), parylene, and combinations thereof.

The expression "complementary pattern" refers to the inverse shape/form of the pattern of interest such as the mold used to cast a metal tool has the inverse form of the tool.

As mentioned the stamp protrusions, when into contact with the support surface, prevent the gel or the gel and microbeads to attach to those areas of the support. The term "protrusion" as used in the present description refers to projections areas of the stamp which are salient in respect to the average surface area of the stamp. These protrusions will eventually lead to patterned regions in the support defined by a complementary pattern of a uniformly distributed gel or by a complementary pattern comprising two or more complementary patterned regions, wherein at least one complementary pattern region is defined by an uniformly distributed gel attached to the support and at least one complementary pattern region is defined by uniformly distributed microbeads "wherein the pattern regions in the support show increased adhesive capacity to cells with respect to the complementary pattern to which the gel or the gel and microbeads are attached".

The term "region" as used herein refers to an area/local/part of the surface of the support which is somehow delimited from another region. In the present invention the attachment of microbeads to the support defines two delimited regions characterized in that one region has reduced adhesive capacity to cells, namely region which has the microbeads attached. In a preferred embodiment the patterned regions have an increased adhesive capacity to cells of at least 0.1 %, at least 0.2 %, at least 0.3 %, at least 0.4 %, at least 0.5 %, at least 0.6 %, at least 0.7 %, at least 0.8 %, at least 0.9 %, at least 1 %, at least 1.1 %, at least 1.2 %, at least 1.3 %, at least 1.4 %, at least 1.5 %, at least 1.6 %, at least 1.7 %, at least 1.8 %, at least 1.9 %, at least 2 % in comparison to the regions with microbeads attached. In another preferred embodiment the patterned regions has an increased adhesive capacity to cells of at least 2 %, at least 3 %, at least 4 %, at least 5 %, at least 6 %, at least 7 %, at least 8 %, at least 9 %, at least 10 %, at least 11 %, at least 12 %, at least 13 %, at least 14 %, at least 15 %, at least 16 %, at least 17 %, at least 18 %, at least 19 %, at least 20 % in comparison to the regions with microbeads attached. In yet another preferred embodiment the patterned region has an increased adhesive capacity to cells of at least 20 %, at least 25 %, at least 30 %, at least 35 %, at least 40 %, at least 45 %, at least 50 %, at least 55 %, at least 60 %, at least 65 %, at least 70 %, at least 75 %, at least 80 %, at least 85 %, at least 90 %, at least 91 %, at least 92 %, at least 93 %, at least 94 %, at least 95 %, at least 96 %, at least 97 %, at least 98 %, at least 99 %, at least 100 % in comparison to the regions with microbeads attached. The adhesive capacity of both regions can be determined by routine experimental procedures, such as, without limitation, seeding a cell solution in the support for a predetermined time, after which the solution is removed and the support is washed with a buffer solution. After washing, the number of cells attached to each region is counted and the reduction between regions proportional to area of said regions calculated.

In a preferred embodiment of the support of the invention the regions in the support onto which the gel or gel and microbeads are attached are further coated with a compound or composition which substantially reduces cell adhesion. The expression "compound or composition which substantially reduces cell adhesion" in the present context refers to blocking agents which reduce the attachment of cells in the regions wherein they are present. Such blocking agents can be further be used in combination with solvents and/or buffers which allow their distribution through the region which is to have a reduced adhesive capacity to cells. Examples of such compounds or composition are, without limitation, bovine serum albumin (BSA), milk powder, casein, polyetilenglycol (PEG), polyacrylamide, phosphatidylcholine, pluronic acids, dextran, self-assembly monolayers of closely packed amphiphilic molecules such as alkanethiols terminated with oligo(ethylene glycol) (OEG) or mixed OEG-OH and OEG-COOH, or alkanethiols terminated in positively charged moieties such as tri(methyl)ammonium (TMA) and negatively charged moieties such as the sulfate group (SO) and polymer brushes (Vaisocherová et al., Anal Bioanal Chem, 2015, 407:3927-3953).

As previously mentioned the complementary patterned may be define by regions of a uniformly distributed gel and uniformly distributed microbeads.

The term "microbeads" as used herein refers to micro-particles that may be spherical or oval or have an irregular shape. The microbeads can be organic, inorganic, synthetic, or natural materials. Examples of said materials are, without limitation, glass, polystyrene, poly(methyl methacrylate), silica, zirconia, titanium, gold, polyethylenepolylactic acid (PLA), poly-L-lactic acid (PLLA), poly glycolic acid (PGA), poly lactic-co-glycolic acid (PLGA) and poly-caprolactone (PCL). In a preferred embodiment of the support of the invention the microbeads are selected from a group consisting of: glass microbeads, polystyrene microbeads, polyacrylamide microbeads, poly(methyl methacrylate) microbeads, silica microbeads, zirconia microbeads, titanium microbeads, gold microbeads, polyethylenepolylactic acid (PLA) microbeads, poly-L-lactic acid (PLLA) microbeads, poly glycolic acid (PGA) microbeads, poly lactic-co-glycolic acid (PLGA) microbeads and poly-caprolactone (PCL) microbeads, preferably glass microbeads, polystyrene microbeads, poly(methyl methacrylate) microbeads, more preferably polystyrene microbeads.

In another preferred embodiment of the support of the invention the microbeads have a diameter of at least 50 nm, at least 100 nm, at least 150 nm, at least 200 nm, at least 250 nm, at least 300 nm, at least 350 nm, at least 400 nm, at least 450 nm, at least 500 nm, at least 550 nm, at least 600 nm, at least 650 nm, at least 700 nm, at least 750 nm, at least 800 nm, at least 850 nm, at least 900 nm, at least 950 nm, at least 1000 nm, at least 2000 nm, at least 3000 nm, at least 4000 nm, at least 5000 nm.

In another preferred embodiment of the support of the invention the microbeads have a diameter of less than 25000 nm, less than 24000 nm, less than 23000 nm, less than 22000 nm, less than 21000 nm, less than 20000 nm, less than 19000 nm, less than 18000 nm, less than 17000 nm, less than 16000 nm, less than 15000 nm, less than 14000 nm, less than 13000 nm, less than 12000 nm, less than 11000 nm, less than 10000 nm, less than 10000 nm, less than 9000 nm, less than 8000 nm, less than 7000 nm, less than 6000 nm, less than 5500 nm.

In a preferred embodiment of the support of the invention the microbeads have a diameter of between 50 nm and 25000 nm. In a preferred embodiment of the support of the invention the microbeads have a diameter of between 50 nm to 1000 nm, between 100 nm to 950 nm, between 150 nm to 900 nm, between 200 nm to 850 nm, between 250 nm to 800 nm, between 300 nm to 750 nm, between 350 nm to 700 nm, between 400 nm to 650 nm, between 450 nm to 600 nm, between 500 nm to 550 nm. In a preferred embodiment of the support of the invention the microbeads have a diameter of between 10000 nm to 25000 nm, between 11500 nm to 23500 nm, between 13000 nm to 22000 nm, between 14500 nm to 20500 nm, between 16000 nm to 19000 nm, between 16500 nm to 17500 nm, between 1000 nm to 10000 nm, between 2000 nm to 9000 nm, between 3000 nm to 8000 nm, between 4000 nm to 7000 nm, between 4000 nm to 6000 nm, between 4000 nm to 5000 nm.

The microbeads present on the surface of the support of the invention are "modified with at least one second functionalizing molecule". Said expression in the context of the present invention refers to the fact that the microbeads comprise a functional group or molecule bound to their surface wherein said functional group or molecule allows the detection and/or interaction of a specific molecule of interest in the medium or in the cell surface of cells that may be present in the support. Examples of second functionalizing molecules are identical to the ones provided for the first functionalizing molecule and the embodiments in relation to the first functionalizing molecule are equally applicable to the second functionalizing molecule.

In a preferred embodiment, the second functionalizing molecule is a high molecular weight compound. In one embodiment, the second functionalizing molecule is a poplypeptide.

In one embodiment, the second functionalizing molecule is a polynucleotide.

In one embodiment, the second functionalizing molecule is a lipid.

Methods for the functionalization of the microbeads are well known in the art. In a preferred embodiment of the support of the invention the molecule is functionalized to the microbeads by Cu(I)-catalyzed azide-alkyne reaction, covalent attachment to amine groups present at the surface, surfactant addition or swelling. In another preferred embodiment of the support of the invention the molecule is attached to the microbeads through a coupling molecular pair, wherein one member of the coupling molecular pair is bound to the microbeads and the other member of the coupling molecular pair is bound to the molecule of interest. The term "coupling molecule pair" as used herein refers to two compounds which have a high affinity for each other producing stable and lasting interactions. Said coupling molecular pair can therefore be used to bind a molecule of interest to the surface of the microbeads. In said case, one of the members of the coupling molecule pair is functionalized to the microbead while the other member of the coupling molecule pair is bound to the molecule of interest. When in close proximity of each other the coupling molecular pair will bind together, effectively functionalizing the microbead with the molecule of interest. Examples of coupling molecular pairs are, without limitation, azide-alkyne, alkaline-nitrone and biotin-streptavidin. In a preferred embodiment of the support of the invention the coupling molecular pair is is the streptavidin biotin molecular pair.

Functionalization of the microbeads with a molecule of interest can also be accomplished with one single coupling molecule which binds both the microbeads and the molecule of interest. Therefore, in a preferred embodiment of the support of the invention the molecule is functionalized through a single coupling molecule. The term "single molecule pair" as used herein refers to a compound which can bind together the surface microbeads and the molecule of interest, producing stable and lasting interactions. Examples of said single coupling molecule are, without limitation, low molecular weight heparin, polyethylene glycol (PEG), 1,4,7,10-tetraazacyclododecane-1,4,7,10-tetraacetic acid (DOTA), diethylene triamine pentaacetic acid (DTPA), silane coupling agents, like aldehyde-, amino-, and hydroxyl- silanes , for example 4-amino butyl triethoxysilane. In a preferred embodiment of the support of the invention the single coupling molecule is selected from a group consisting of: low molecular weight heparin, polyethylene glycol (PEG), 1,4,7,10-tetraazacyclododecane-1,4,7,10-tetraacetic acid (DOTA), diethylene triamine pentaacetic acid (DTPA), aldehyde-silanes, amino-silanes, hydroxyl-silanes or any combination thereof.

In a preferred embodiment of the support of the invention the microbeads attached to the surface comprise microbeads with at least one second functionalizing molecule and microbeads which are not functionalized, wherein the microbeads which are not functionalized may be functionalized while attached to the surface of the support. In another preferred embodiment of the support of the invention the microbeads attached to the surface comprise at least two types of microbeads wherein the each type of microbeads is modified with at least one second functionalizing molecule, wherein the functionalizing molecules are identical or different for each type of microbeads. In another preferred embodiment of the support of the invention the microbeads attached to the surface comprise at least two types of microbeads wherein the each type of microbeads is modified with at least one second functionalizing molecule, wherein the functionalizing molecules are distinct for each type of microbeads and each type of microbeads is of the same material or of a different material or a combination thereof.

The support comprises at least two types of regions, one of which has an increased adhesive capacity to cells in respect to the other region. The regions of the support which have gel or gel and microbeads attached to, are defined by the complementary pattern of the patterned regions, as previously mentioned. In a preferred embodiment of the support of the invention the patterned regions of the support which do not have microbeads attached to have a geometric form selected from a group consisting of: triangle, square, rectangle, rhombus, parallelogram, trapezoid, trapezium, n-agon, circle, ellipse or any combination thereof. In a more preferred embodiment of the support of the invention the patterned regions of the support have a geometrical form, preferably a circular form with the diameter of between 10 micrometers (µm) to 200 µm, preferably from 50 µm to 100 µm.

In a preferred embodiment of the support of the invention, the patterned regions in the support are coated with a compound or composition that promotes cell adhesion. In another preferred embodiment of the support of the invention, the coating of the support with a compound or composition that promotes cell adhesion is carried out by coating the protrusions of the stamp with said compound or composition that promotes cell adhesion and contacting the support with the stamp thereby allowing the transfer of the compound or composition from the stamp onto the support with a pattern that matches the pattern of protrusions of the stamp.

The expression "compound or composition which promotes cell adhesion" in the context of the present description refers to a protein located on the cell surface involved in the process of cell-cell or cell-extracellular matrix binding. The cell adhesion proteins includes immunoglobulins, integrins, cadherins and selectins. Non-limiting examples of compounds which promote cell adhesion are, without limitation, collagen types I, II and IV, elastin, fibronectin, vitronectin and laminin. In a preferred embodiment of the support of the invention the compound that promotes cell adhesion is an extracellular matrix molecule selected from the group which consists of: collagen type I, collagen type II, collagen type IV, elastin, fibronectin, vitronectin, laminin or any combination thereof, preferably fibronectin.

The term "extracellular matrix" or its acronym "ECM" as used herein refers to the non-cellular component present within all tissues and organs, which provides not only essential physical scaffolding for the cellular constituents but also initiates crucial biochemical and biomechanical cues that are required for tissue morphogenesis, differentiation and homeostasis.

In the context of the present invention the expression "allowing the transfer of the compound or composition from the stamp onto the support with a pattern that matches the pattern of protrusions of the stamp" refers to process of binding, joining or connecting said compound or composition to the surface of the support in such a way as to form a pattern in the support which will determine the regions within the support of the invention onto which the cells will adhere. Said process can be done by a variety of methods which the skilled person in the art will be knowledgeable about, collectively known as soft-lithography, such as micro-contact printing, micro-molding in capillary, replica molding, solvent-assisted micro-molding, phase-shifting edge lithography, decal transfer lithography, nanotransfer printing, dip-pen lithography, nano-skiving and vacuum-drive soft lithography. In a preferred embodiment of the support of the invention the patterned regions are created by imprinting the support with stamps containing the compound or composition which promotes cell adhesion using a technique selected from the group consisting of: micro-contact printing, micro-molding in capillary, replica molding, solvent-assisted micro-molding, phase-shifting edge lithography, decal transfer lithography, nanotransfer printing, dip-pen lithography, nano-skiving and vacuum-driven soft lithography, preferably microcontact printing, preferably vacuum-driven soft lithography. In another preferred embodiment of the support of the invention the patterned regions are created using vacuum-driven soft lithography.

As used herein, the term "microcontact printing" is meant to designate any process useful for applying a molecule onto a surface, preferably such that the dimensions of the printed structures lie in the µm-range and/or in the nm-range. In a preferred embodiment of the support of the invention the patterned regions are created by microcontact printing with the use of stamps comprising a elastomer selected from a group consisting of: PDMS, PDMA, polyurethane rubber, styrene butadiene rubber, acrylonitrile butadiene rubber, poly-cis-isoprene, thermoplastic polyurethane, thermoplastic copolyester, thermoplastic polyamide, SU-8, polyimides, polyurethanes, polyamides, PLGA, PLA, PGA, PCL, PGS, chitosan, parylene or any combination thereof, preferably PDMS.

In a preferred embodiment of the support of the invention the support further contains viable cells which are attached to the support or to the molecules within the support which promote cell adhesion. The term "viable cells" as used herein refers to cells which are capable of proliferate in a suitable medium as well as cells which are capable of maintaining a basal metabolism suitable for their functions without dividing, indicative of a healthy cell state. In a preferred embodiment of the support of the invention the support further contains cells, wherein the cells are insect cells, avian cells, mammalian cells, hybridoma cells, primary cells, continuous cell lines, stem cells and/or genetically engineered cells, and wherein the genetically engineered cells are recombinant cells expressing a heterologous protein or polypeptide. In another preferred embodiment of the support of the invention, the support further contains animal cells, wherein the animal cells are mammalian cells, preferably selected from a group consisting of: BSC-1 cells, LLC-MK cells, CV-1 cells, CHO cells, COS cells, murine cells, human cells, HeLa cells, 293 cells, VERO cells, MDBK cells, MDCK cells, MDOK cells, CRFK cells, RAF cells, TCMK cells, LLC-PK cells, PK15 cells, WI-38 cells, MRC-5 cells, T-FLY cells, BHK cells, SP2/0 cells, NSO. perC6 (human retina cells) or derivatives thereof. In another preferred embodiment of the support of the invention, the support further contains insect cells, wherein the insect cells are selected from a group consisting of: Sf21, Sf9, and the BTI-TN-5B1-4 (or High Five) cells, or any combination thereof.

The term "avian cells" as used herein refers to cells or cell cultures with cells originated from birds. A list of acceptable lines which can be used in the context of the present invention is available in the literature (K. Nazerian, 1987, Avian Pathology, 16:3, 527-544).

The term "insect cells" as used herein refers to cells originated from insects, preferably lepidopteran cell lines. A list of available cell line from lepidopteran is available (Lynn D., 2007, pp. 118 - 129, In: Murhammer D.W. (Eds) Baculovirus and Insect Cell Expression Protocols. Methods in Molecular Biology™, vol 388. Humana Press. In another preferred embodiment of the support of the invention the cells are insect cells, wherein the insect cells are selected from a group consisting of: Sf21, Sf9, and the BTI-TN-5B1-4 (or High Five) cells, or any combination thereof.

The term "hybridoma cell" as used herein refers to a hybrid cell obtained from the fusion of an antibody producing B-cell of interest and an immortal B cell cancer cell, a myeloma. These cells allow the production of antibodies of interest, monoclonal antibodies.

The term "primary cell" in the context of the present description refers to cells freshly obtained from a multicellular organism which are culture ex vivo.

The term "stem cell" as used herein refers to an undifferentiated cell that has the ability to divide and replicate itself for an indefinite period while maintaining an undifferentiated state even ex vivo. Furthermore, the stem cell has the ability to differentiate into many kinds of different cells in an organism depending on the developmental stage and location of an individual. Stem cells can be totipotent, pluripotent or multipotent.

In another preferred embodiment of the support of the invention the support further contains stem cells, preferably induced stem cells, more preferably mesenchymal stem cells (MSC). The term "mesenchymal stem cells" or its acronym "MSC" as used herein refers to mesenchymal and/or adipose stem cells. In a preferred embodiment of the support of the invention the support comprises MSC cells selected from a group consisting of: bone-marrow MSCs, Adipose MSCs, Umbilical Cord MSCs, ES Cell Derived MSCs and hair follicle derived MSCs, preferably hair follicle derived MSCs.

### Method for the production of the support of the invention

Another aspect of the present invention relates to a method for producing the support of the invention, from here onwards the producing method of the invention, selected from:
(a) a method comprising the steps of:
   (i) Providing a support;
   (ii) Contacting the support with a stamp, said stamp having protrusions which define the desired pattern in the support, wherein the contact of the support with the stamp forms bottomless channels between the support and the stamp,
   (iii) contacting the support along the bottomless channels with a gel solution and allowing the gel solution to distribute uniformly in those regions in the support which are not protected by the protrusions of the stamp, and
   (iv) polymerizing the gel in order for the gel to adhere to the support as to obtain a uniform attachment onto those regions in the support which are not protected by the protrusions of the stamp obtaining a pattern defined by the gel which is complementary to the desired pattern,
   wherein the gel solution comprises at least one first functionalizing molecule or at least one first functionalizing molecule is added to the gel after the gel is polimerized;
   or
(b) a method comprising the steps of:
   (i) Providing a support;
   (ii) Contacting the support with a stamp, said stamp having protrusions which define the desired pattern in the support, wherein the contact of the support with the stamp forms bottomless channels between the support and the stamp,
   (iii) contacting the support along the bottomless channels with a gel solution and a microbead suspension wherein the microbeads are modified with at least one second functionalizing molecule and allowing the microbeads to adhere to the support as to obtain a uniform deposition in and attachment of the microbeads onto those regions in the support which are not protected by the protrusions of the stamp nor occupied by the gel solution, and
   (iv) and polymerizing the gel in order for the gel to adhere to the support as to obtain a uniform attachment onto those regions in the support which are not protected by the protrusions of the stamp nor occupied by the microbeads, obtaining a pattern defined by the gel and microbeads which is complementary to the desired pattern,

wherein the gel comprises at least one first functionalizing molecule and/or the microbeads are modified with at least one second functionalizing molecule and
wherein the gel solution comprises at least one first functionalizing molecule or at least one first functionalizing molecule is added to the gel after the gel is polymerized.

All the previous defined terms and expressions are equally valid for the present aspect and its embodiments.

In a first step, for both method (a) and (b), a support is provided. In some embodiments, the support of step (i) is made from a material selected from a group consisting of: glass, borosilicate glass, quartz glass, polystyrene, polymethylmethacrylate (PMMA), polycarbonate and polyethylene, preferably glass.

In a second step, step (ii) for both methods (a) and (b) according to the present invention, involves contacting the support with a stamp, said stamp having protrusions which define a desired pattern, wherein the contact of the support with the stamp forms bottomless channels between the support and the stamp.

The expression "contact of the support with the stamp" as used herein refers to the process of bringing the support and the stamp into very close contact thereby allowing the protrusions of the stamp to touch the surface of the support. This sandwich type process, wherein the support and the stamp are place onto each other prevents the gel and the microbeads from attaching to the support surface in those regions where the protrusions touch the surface of the support.

In a preferred embodiment of the producing method of the invention the areas of the support which are in contact with the stamp in step (ii) of methods (a) and (b) are coated with a compound or composition that promotes cell adhesion. In another preferred embodiment of the producing method of the invention the protrusions of the stamp in step (ii) of methods (a) and (b) are coated with a compound or composition that promotes cell adhesion thereby allowing the transfer of the compound or composition from the stamp onto the support with a pattern that matches the pattern of protrusions of the stamp.

In a preferred embodiment of the producing method of the invention the stamp of step (ii) of methods (a) and (b) is made of an elastomer selected from a group consisting of: PDMS, polyurethane rubber, styrene butadiene rubber, acrylonitrile butadiene rubber, poly-cis-isoprene, thermoplastic polyurethane, thermoplastic copolyester, thermoplastic polyamide, SU-8, polyimides, polyurethanes, polyamides, PLGA, PLA, PGA, PCL, PGS, chitosan, parylene or any combination thereof, preferably PDMS. In a preferred embodiment of the producing method of the invention the stamp is a PDMS stamp.

In a preferred embodiment, step (ii) of methods (a) and (b) is carried out by vacuum-driven soft lithography. In some embodiments, step (ii) is carried out by applying pressure onto the stamp or onto the support or onto both the stamp and the support. In some embodiments, the pressure is achieved by creating vacuum in the chamber formed between the support and the stamp. When the chamber is provided with operable inlet(s) and an operable outlet(s), all outlets are closed so as to allow the generation of vacuum in the chamber.

In a preferred embodiment of the producing method of the invention the patterned regions of the support have a geometric form selected from a group consisting of: triangle, square, rectangle, rhombus, parallelogram, trapezoid, trapezium, n-agon, circle, ellipse or any combination thereof. In a more preferred embodiment of the support of the invention the patterned regions are circles with the diameter between 10 micrometers (µm) to 200 µm, preferably from 50 µm to 100 µm. In a preferred embodiment of the producing method of the invention the patterned regions of the support are according to Figure 5.

In the present producing method of the invention the attachment of the gel or gel and microbeads to the support defines two delimited regions, the patterned regions and the complementary regions, characterized in that the latter region has reduced adhesive capacity to cells. In a preferred embodiment of the producing method of the invention the regions in the support onto which the microbeads attaches in step (iii) of method (b) and onto which the gel attaches in step (iv) of method (a) and (b) are further coated with a compound or composition which substantially reduces cell adhesion.

In a preferred embodiment the complementary region with gel or the complementary regions with gel and microbeads attached have a reduced adhesive capacity to cells of more than at least 0.1 %, at least 0.2 %, at least 0.3 %, at least 0.4 %, at least 0.5 %, at least 0.6 %, at least 0.7 %, at least 0.8 %, at least 0.9 %, at least 1 %, at least 1.1 %, at least 1.2 %, at least 1.3 %, at least 1.4 %, at least 1.5 %, at least 1.6 %, at least 1.7 %, at least 1.8 %, at least 1.9 %, at least 2 % less than the patterned regions. In another preferred embodiment the complementary regions have a reduced adhesive capacity to cells of more than at least 2 %, at least 3 %, at least 4 %, at least 5 %, at least 6 %, at least 7 %, at least 8 %, at least 9 %, at least 10 %, at least 11 %, at least 12 %, at least 13 %, at least 14 %, at least 15 %, at least 16 %, at least 17 %, at least 18 %, at least 19 %, at least 20 % less than the patterned regions. In yet another preferred embodiment the complementary region has a reduced adhesive capacity to cells of more than at least 20 %, at least 25 %, at least 30 %, at least 35 %, at least 40 %, at least 45 %, at least 50 %, at least 55 %, at least 60 %, at least 65 %, at least 70 %, at least 75 %, at least 80 %, at least 85 %, at least 90 %, at least 91 %, at least 92 %, at least 93 %, at least 94 %, at least 95 %, at least 96 %, at least 97 %, at least 98 %, at least 99 %, at least 100 % than the patterned regions.

In a preferred embodiment of the producing method of the invention the protrusions of the stamp of step (ii) of methods (a) and (b) are coated with a compound or composition which promotes cells adhesion which will be transferred to the support coating the patterned regions. In a preferred embodiment of the producing method of the invention the compound or composition which promotes cell adhesion of step (ii) of methods (a) and (b) is an extracellular matrix molecule selected from the group which consists of: collagen type I, collagen type II, collagen type IV, elastin, fibronectin, vitronectin, laminin or any combination thereof, preferably fibronectin.

In some embodiments, the stamp and the support are placed so that at least one chamber is formed between these two elements, said chamber being provided with a least one operable inlet. In some embodiments, the stamp and the support are placed so that at least two chambers are formed between these two elements, said chambers being independent from each other and each chamber provided with a least one operable inlet. In some embodiments, the stamp and the support are placed so that at least one chamber is formed between these two elements, said chamber being provided with a least one operable inlet and at least one operably outlet. In some embodiments, the stamp and the support are placed so that at least two chambers are formed between these two elements, said chambers being independent from each other and each chamber provided with a least one operable inlet and at least one operable outlet. In some embodiments, the stamp and the support are placed so that at least one, two, three, four, five, six, seven, eight, nine, ten or more chambers are formed between these two elements, said chambers being independent from each other and each chamber provided with a least one operable inlet and optionally one outlet.

Step (iii) of the method for the production of the support of the invention refers to the deposition and polymerization of a gel solution or the deposition of microbeads in a complementary pattern or complementary pattern regions onto the surface of the support, wherein said deposition is made by allowing the gel and beads to flow along the bottomless channels formed in between the stamp and the support, wherein said bottomless channels are present due to the fact that the protrusions present in the stamp which form contact zones in step (ii) of the producing method of the invention are elevated in height in relation to the channels. Step (iii) allows the uniform or homogeneous deposition of gel and microbeads in a complementary pattern onto the surface of the support. The term "bottomless channels" as used herein refers to the fact the said channels are opened at both extremes, therefore, lacking a base or bottom.

In some embodiments of the producing method of the invention, in step (iii) of method (a), the chamber formed between the stamp and the support is provided with at least one operable inlet and said step (iii) is carried out by injecting a gel solution through the inlet, maintaining the gel solution within the channels for a sufficient time to allow uniform deposition of the gel on the support and polymerizing the gel.

In a particular embodiment in step (iii) of method (b) of the producing method of the invention, the gel solution is distributed through bottomless channels which form an independent chamber and the microbeads suspensions is distributed through through bottomless channels which form another independent chamber, wherein the gel solution and the microbead suspension do not come into contact with one another.

In some embodiments of the producing method of the invention, in step (iii) of method (b), the independent chambers formed between the stamp and the support are provided with at least one operable inlet each, and step (iii) is carried out by injecting gel solution through one of the inlets and microbead suspension through another of the inlets, maintaining the gel solution and the microbead solution within the channels for sufficient time to allow the uniform deposition of the gel and of the microbeads onto the support. In a preferred embodiment of the producing method of the invention, in step (iii) of method (b), once the microbeads are deposited uniformly the suspension is removed by the inlet.

In some embodiments of the producing method of the invention, in step (iii) of method (b), the independent chambers formed between the stamp and the support are provided with at least one operable inlet each and at least one outlet each, and step (iii) is carried out by injecting gel solution through one of the inlets and microbead suspension through another of the inlets, while the outlets are is sealed, maintaining the gel solution and the microbead solution within the channels for sufficient time to allow the uniform deposition of the gel and of the microbeads onto the support and extracting the suspension through the outlet.

In some embodiments of step (iii) of method (a) and (b) the contacting the support along the bottomless channels with a gel solution is carried out by injecting a gel solution at a temperature wherein the gel is liquefied through the at least one inlet maintaining the gel solution within the channels for a sufficient time so as to obtain a uniform distribution of the gel on the support before proceeding with step (iv). In another preferred embodiment of step (iii) of method (a) and (b) the the contacting the support along the bottomless channels with a gel solution and a microbead suspension is carried out by injecting a gel solution wherein the gel solution is liquefied through the at least one inlet and by injecting a suspension of microbeads through the at least one inlet, maintaining the gel solution and the microbead suspension within the channels for a sufficient time so as to obtain a uniform distribution of the gel and a uniform deposition of the microbeads on the support before proceeding with step (iv).

The term "liquefied" as used herein refers to a material which is in a liquid state or in a fluid state as to allow the material to flow through the bottomless channels. The material may be force into a liquid state by changing its temperature, either increasing it or decreasing it, or by dissolving it in a solvent such as water.

In some embodiments of step (iii) of method (a) and (b) the polymerization is carried out by changing the modifying the conditions of the gel solution to conditions which promote the polymerization of the gel. As the expert in the field will be aware, several gel compounds and compositions are known in the art, and each of them requires different techniques for polymerization, such as temperature shifts from higher temperatures to lower temperatures for collagen based gels for example, temperature shifts from lower temperatures to higher temperatures for extracellular matrix components based gels such as Matrigel^{©} for example, photocatalysm for gelatin methacrylate for example, as previously defined.

In some embodiments of step (iii) of method (a) and (b) the polymerization is carried out by UV irradiation, wherein the UV irradiation is carried out with a wavelength of between 300 nm and 450 nm, preferably 350 nm and 400 nm. In another particular embodiment gel is polymerized with UV irradiation with a wavelength of between 300 nm and 450 nm, preferably 350 nm and 400 nm, wherein the irradiation is carried out in cycles, each cycle consisting of 5 seconds (s) of irradiation (ON) followed by 30 seconds without irradiation (OFF), for between 5 to 70 cycles, preferably for 10, 15, 20, 25, 30, 35, 40, 45, 50, 55, 60, 65 cycles.

In another particular embodiment gel is polymerized with UV irradiation with a wavelength of between 300 nm and 450 nm, preferably 350 nm and 400 nm, wherein the irradiation is carried out continuously for between 30 seconds (s) and 20 minutes (m), preferably for 1 m, 2 m, 3 m, 4 m, 5 m, 6 m, 7 m, 8 m, 9 m, 10 m, 11 m, 12 m, 13 m, 14 m, 15 m, 16 m, 17 m, 18 m, 19 m.

In some embodiments of step (iii) of method (a) and (b) the polymerization is carried out by increasing the temperature in order to promote the polymerization of the gel solution. In some embodiments of step (iii) of method (a) and (b) the polymerization is carried out by decreasing the temperature in order to promote the polymerization of the gel solution.

In some embodiments of step (iii) of method (a) and (b) the polymerization is carried out by increasing the temperature to between about 35°C to about 39°C, preferably about 36°C, 37°C or 38°C. In some embodiments of the producing method of the invention, in step (iii) of method (a), the chamber formed between the stamp and the support is provided with at least one operable inlet and at least one operable outlet, said step (iii) is carried out by injecting a gel solution through the at least one inlet while the at least one outlet is closed, maintaining the gel solution within the channels for a sufficient time to allow uniform deposition of the gel on the support and polymerizing the gel. In some embodiments of step (iii) of method (a) the polymerization is carried out by UV irradiation or by increasing the temperature to between about 35°C to about 39°C, preferably about 36°C, 37°C or 38°C.

In some embodiment of step (iii) of method (b) of the producing method of the invention the suspension containing microbeads is allowed to flow for 1 min to 10 minutes, preferably 2 min to 8 min, more preferably for 5 min. In some embodiment of method (a) and (b) the suspension containing microbeads or the gel solution is allowed to flow until the bottomless channels are filled. In some embodiment the suspension containing microbeads or the gel solution is allowed to flow until the outlet is filled. In some embodiment of method (a) and (b) the outlet is unsealed after the suspension containing microbeads has filled the outlet and the suspension is allowed to flow out of the outlet.

In some embodiments step (iii) of method (a) and (b) of the producing method of the invention is carried out while pressure is being applied to bring into contact the slab and the support. In some embodiments the pressure is achieved by creating vacuum in the chamber formed between the support and the slab. In some embodiments, the gel solution or the microbead suspension containing microbeads is placed in the inlet allowing the suspension to enter the channels due to the negative pressure therein as a result of the vacuum applied in step (ii), wherein if the chamber comprises an outlet said outlet is sealed while the gel solution or the suspension containing microbeads is placed in the inlet.

In a preferred embodiment of the producing method of the invention in step (iii) and step (iv) of the method (a) and (b) the gel solution or the gel solution and microbead suspension further comprises a compound or composition which substantially reduces cell adhesion or a compound of composition of interest. In another preferred embodiment of the producing method of the invention the regions in the support onto which the gel is attached to in step (iv) of method (a) and (b) or onto which the microbeads are attached in step (iii) of method (a) and (b) are further coated with a compound or composition which substantially reduces cell adhesion. Examples of said compounds or compositions were provided above and said examples are equally valid for the present method of the invention.

In a preferred embodiment of the producing method of the invention the gel of step (iii) of methods (a) and (b) is a hydrogel or an ionogel. In another preferred embodiment of the producing method of the invention the gel of step (iii) of methods (a) and (b) is a hydrogel and comprises a polymer selected from a group consisting of: alginic acid, agarose, hyaluronic acid, polyethylene glycol, gelatin, collagen, fibrin, chitosan, fibrin, fibrinogen, fibronectin, laminin, elastin, and combinations thereof. In yet another preferred embodiment of the gel of the invention the gel of step (iii) of methods (a) and (b) is a hydrogel wherein the polymer is gelatin, preferably gelatin methacrylate.

In a preferred embodiment of the producing method of the invention the gel of step (iii) of methods (a) and (b) is an ionogel comprising an ionic liquid selected from the group consisting of: 1-methyl-3-butylimidazole tetrafluoroborate, 1-methyl-3-butylimidazole hexafluorophosphate, and 1-ethyl-3-methylimidazole ester Acid salt, 1-methyl-butylimidazole hydrogen sulfate, 1-ethyl-3-methylimidazole bistrifluoromethanesulfonimide salt, 1-ethyl-3-methylimidazole dicyanamide 1-ethyl-3-methylimidazolium ethyl sulphate, trihexyltetradecylphosphonium dicyanamide, choline acetate, 1-butyl-2,3-dimethylimidazolium, 1-butyl-3-methylimidazolium, 1-butyl-1-methylpyrrolidinium, 1-butyl-1-methylpiperidinium, and methyltrioctylammonium.

In a preferred embodiment of the producing method of the invention the complementary pattern defined by the gel has a height of between 1 µm and 200 µm.

In a preferred embodiment of the producing method of the invention the microbeads of step (iii) of method (b) have a diameter between 50 nm and 25000 nm. In a preferred embodiment of the producing method of the invention the microbeads of step (iii) of method (b) are selected from a group consisting of: glass microbeads, polystyrene microbeads, polyacrylamide microbeads, poly(methyl methacrylate) microbeads, silica microbeads, zirconia microbeads, titanium microbeads, gold microbeads, polyethylenepolylactic acid (PLA) microbeads, poly-L-lactic acid (PLLA) microbeads, poly glycolic acid (PGA) microbeads, poly lactic-co-glycolic acid (PLGA) microbeads and poly-caprolactone (PCL) microbeads, preferably polystyrene microbeads.

In some embodiments, after the gel or the gel and microbeads containing the first functionalizing molecule and/or the second functionalizing molecule are deposited on the support, the support is then contacted with a quenching agent that reduces non-specific binding of any other molecule that is contacted with the support. Suitable quenching agents that can be used include, without limitation, milk powder, bovine serum albumin, heat-inactivated serum or a combination of one or more of the above.

As stated in the producing method of the invention the gel solution can comprise a first functionalizing molecule when is inserted in step (iii) of methods (a) and (b) or said first functionalizing molecule can be added after the gel is polymerized. In a preferred embodiment of the support of the invention the gel comprises at least two first functionalizing molecules, wherein at least one first functionalizing molecule is present when the gel is polymerized and at least another first functionalizing molecule is added to the gel after the gel polymerization.

In a preferred embodiment of the producing method of the invention the solution of microbeads of step (iii) of method (b) comprises microbeads with at least one functionalizing molecule and microbeads which are not functionalized, wherein the microbeads which are not functionalized may be functionalized while attached to the surface of the support.

In some embodiments, the producing method of the invention further comprises contacting the support with a cell population under conditions adequate for the binding of the cells to the support through interactions between the cells and the compound in the support that promotes cell adhesion and the formation of patterned cell clusters.

The term "cell population" as used herein is synonymous with the term "cell culture". In a preferred embodiment of the producing method of the invention the cell population contacted with the support is selected from a group consisting of: insect cells, avian cells, mammalian cells, hybridoma cells, primary cells, continuous cell lines, stem cells and/or genetically engineered cells, and wherein the genetically engineered cells are recombinant cells expressing a heterologous protein or polypeptide.

In another preferred embodiment of the producing method of the invention the cell population contacted with the support is an animal cell population, wherein the animal cells are mammalian cells, preferably selected from a group consisting of: BSC-1 cells, LLC-MK cells, CV-1 cells, CHO cells, COS cells, murine cells, human cells, HeLa cells, 293 cells, VERO cells, MDBK cells, MDCK cells, MDOK cells, CRFK cells, RAF cells, TCMK cells, LLC-PK cells, PK15 cells, WI-38 cells, MRC-5 cells, T-FLY cells, BHK cells, SP2/0 cells, NSO. perC6 (human retina cells) or derivatives thereof.

In another preferred embodiment of the producing method of the invention the cell population contacted with the support is an insect cell population, wherein the insect cells are selected from a group consisting of: Sf21, Sf9, and the BTI-TN-5B1-4 (or High Five) cells, or any combination thereof.

In another preferred embodiment of the effect method of the invention the cell population contacted with the support is a stem cell population, preferably mesenchymal stem cells (MSC), wherein the MSC cells are selected from a group consisting of: bone-marrow MSCs, Adipose MSCs, Umbilical Cord MSCs, ES Cell Derived MSCs and hair follicle derived MSCs, preferably hair follicle derived MSCs.

In order to contact the support with a cell population a structure which maintains the cell population as well as its adequate medium may be required. In a preferred embodiment of the producing method of the invention the cell population contacting the support is placed inside a well-like structure wherein said well-like structure is formed by a base and a vertical wall or walls, wherein the base is formed by the support layer comprising the predetermined patterns base.

The term "well-like structure" in the present context refers to an enclosure or compartment formed by a base, a vertical wall or walls and an open top. Said base is the support which contains the patterned regions onto which the vertical wall or walls are placed or in which the wall or walls are assembled, creating a seal between the base and the vertical wall or walls, said seal allowing to retain liquids or solutions which are required for the viability of the cell population placed in contacted with the support. The term "well-like structure" does not imply any shape or form of said structure, as the functional effect required by the structure can be achieved by any shape or form of the walls, such as one single circular wall or four walls forming a square. Furthermore, the well-like structure vertical walls can be preassembled and placed onto the support, such as, without limitation, bottomless wells formed by PDMS/PDMA slabs which are placed onto the support, or be assembled directly on the support, such as, without limitation, hydrogels or silicone which can be placed onto the support in liquid form and allowed to solidify to form the well-like structure. In addition, as the skilled person in the field will be aware, materials may be required to seal the well-like structure, and said materials are well known in the field, with the only consideration of use being an inert material in relation to the cell population. Furthermore, the well-like structure may be treated agents in order to saturate and/or block protein-binding sites on the walls as to prevent unwanted interactions between the cells and the structure. Said agents are well known by the skilled person in the field and part of routine experimentation work. Examples include, without limitation, bovine serum albumin (BSA) and powder milk.

In a preferred embodiment of the producing method of the invention the cell population is placed in contact with the support inside a well-like structure wherein said well-like structure comprises vertical walls made from a material selected from a group consisting of: polydimethylsiloxane (PDMS), glass, poly(methyl methacrylate) (PMMA), pressure sensitive adhesive sheets (PSA), cyclic olefin copolymer (COC), cyclic olefin polymers (COP), polyethylene terephthalate (PET), BioMed Clear Resin, polyether ether ketone (PEEK), polystyrene (PS), polypropylene (PP), polyethylene (PE), polycarbonate or any combination thereof.

The term BioMed Clear Resin refers to the commercially available hard resin from FormLabs (https://formlabs-media.formlabs.com/datasheets/2001432-TDS-ES-0.pdf])

### Method for determining the effect of a molecule in the cell culture

Another aspect of the present invention relates to a method for determining effect of a molecule on a cell population, from here onwards the effect method of the invention, which comprises the steps of:
(i) Providing the support of the invention according to the invention wherein said support contains the cell population wherein the cells of the population are attached to the support and wherein the molecule, the effect of which on the cell population is to be determined is the at least one first functionalizing molecule and/or the at least one second functionalizing molecule;
(ii) Maintaining the support under adequate conditions to allow the at least one first functionalizing molecule and/or the at least one second functionalizing molecule to exert its effect on the cells of the cell population; and
(iii) Detecting the response of the cells within the cell population to said first functionalizing molecule and/or the at least one second functionalizing molecule,
wherein the detection of a response of the cells to the at least one first functionalizing molecule and/or the at least one second functionalizing molecule is indicative that the at least one first functionalizing molecule and/or the at least one second functionalizing molecule exerts an effect on the cells of the cell population.

All the previous defined terms and expressions are equally valid for the present aspect and its embodiments.

In a first step, the method for determining the effect of a molecule in the cell culture comprises providing a support according to the invention wherein said support contains the cell population wherein the cells of the population are attached to the support by adhesion to the compound promoting cell adhesion and wherein the functionalizing molecule is the molecule of which the effect on the cell population is to be determined.

In a preferred embodiment of the effect method of the invention the cell population in step (i) is selected from a group consisting of: insect cells, avian cells, mammalian cells, hybridoma cells, primary cells, continuous cell lines, stem cells and/or genetically engineered cells, and wherein the genetically engineered cells are recombinant cells expressing a heterologous protein or polypeptide.

In another preferred embodiment of the effect method of the invention the cell population in step (i) is an animal cell population, wherein the animal cells are mammalian cells, preferably selected from a group consisting of: BSC-1 cells, LLC-MK cells, CV-1 cells, CHO cells, COS cells, murine cells, human cells, HeLa cells, 293 cells, VERO cells, MDBK cells, MDCK cells, MDOK cells, CRFK cells, RAF cells, TCMK cells, LLC-PK cells, PK15 cells, WI-38 cells, MRC-5 cells, T-FLY cells, BHK cells, SP2/0 cells, NSO. perC6 (human retina cells) or derivatives thereof.

In another preferred embodiment of the effect method of the invention the cell population is step (i) is an insect cell population, wherein the insect cells are selected from a group consisting of: Sf21, Sf9, and the BTI-TN-5B1-4 (or High Five) cells, or any combination thereof.

In another preferred embodiment of the effect method of the invention the cell population is step (i) is a stem cell population, preferably mesenchymal stem cells (MSC), wherein the MSC cells are selected from a group consisting of: bone-marrow MSCs, Adipose MSCs, Umbilical Cord MSCs, ES Cell Derived MSCs and hair follicle derived MSCs, preferably hair follicle derived MSCs.

In a preferred embodiment of the effect method of the invention the cells are attached to the support by a compound which promotes cell adhesion of step (i), wherein said compound is an extracellular matrix molecule selected from the group which consists of: collagen type I, collagen type II, collagen type IV, elastin, fibronectin, vitronectin, laminin or any combination thereof, preferably fibronectin.

In a preferred embodiment of the effect method of the invention the at least one first functionalizing molecule and/or the at least one second functionalizing molecule is selected from a group consisting of growth factors, differentiation factors, cell adhesion molecules or proteins, pharmaceutical small molecules and any combination thereof.

In another preferred embodiment of the effect method of the invention the at least one first functionalizing molecule and/or the at least one second functionalizing molecule of step (i) is selected from a group consisting of: growth factors, differentiation factors, cell adhesion molecules or proteins, cytokines, pharmaceutical small molecules and any combination thereof.

The term "growth factor" as used herein refers to any molecule or protein that specifically stimulates target cells to proliferate, differentiate, or alter their function or phenotype. In another preferred embodiment of the effect method of the invention the at least one first functionalizing molecule and/or the at least one second functionalizing molecule of step (i) is a growth factor selected from the group consisting of: vascular endothelial growth factor (VEGF), collagen, bone morphogenic factor-β, epidermal cell growth factor (EGF), platelet derived growth factor (PDGF), nerve growth factor (NGF), fibroblast growth factor (FGF), insulin-like growth factor (IGF), transforming growth factor (TGF) and any combination thereof.

The term differentiation factor as used herein refers to a molecule that promotes the differentiation of cells. In another preferred embodiment of the effect method of the invention the at least one first functionalizing molecule and/or the at least one second functionalizing molecule of step (i) is a differentiation factor selected from the group consisting of: neurotrophin, colony stimulating factors (CSF), transforming growth factor (TGF) and any combination thereof.

As used herein, the term "adhesive molecule or protein" or "cell adhesion molecule or protein" refers to a molecule or protein that promotes attachment of a cell to a bead and/or fibril. In preferred embodiment of the effect method of the invention the at least one first functionalizing molecule and/or the at least one second functionalizing molecule of step (i) is an adhesion molecule protein selected from the group consisting of: integrins, cadherins, selectins and any combination thereof.

The term "cytokine" is a generic term for proteins released by one cell population that act on another cell population as intercellular mediators. Examples of such cytokines are lymphokines, monokines, and traditional polypeptide hormones. Included among the cytokines are growth hormone include, such as human growth hormone, N-methionyl human growth hormone, and bovine growth hormone; parathyroid hormone; thyroxine; insulin; proinsulin, relaxin, prorelaxin, glycoprotein, hormones such as follicle stimulating hormone (FSH), thyroid stimulating hormone (TSH), and luteinizing hormone (LH), hepatocyte growth factor, fibroblast growth factor, prolactin, placental lactogen, factor-α and tumor growth-β; Mullerian inhibiting substance, gonadotropin-associated peptide mouse, inhibin, activin, vascular endothelial growth factor, integrin, thrombopoietin (TPO), nervous factors, such as EGF-β growth, platelet growth factor, transforming growth factors (TGF) such as TGF-α and TGF-β, insulin-like factor-I and -II growth, Erythropoietin (EPO), osteoinductive factors, interferons, such as interferon-α, interferon-β, and interferon-γ, colony stimulating factors (CSF) such as macrophage-CSF (M-CSF), CSF granulocyte-macrophage (GM-CSF), and granulocyte-CSF (G-CSF), interleukins (ILs) such as IL-1, IL-1a, IL-2, IL-3, IL-4, IL-5, IL-6, IL-7, IL-8, IL-9, IL-11 , IL-12; tumor necrosis factor, TNF-α as TNF-β ; and other polypeptide factors including LIF and kit ligand (KL). As used herein, the term cytokine includes proteins from natural sources or from recombinant cell culture and biologically active equivalents of the native sequence cytokines. In a preferred embodiment of the effect method of the invention the at least one first functionalizing molecule and/or the at least one second functionalizing molecule of step (i) is selected from a group consisting of: human growth hormone, N-methionyl human growth hormone, bovine growth hormone, parathyroid hormone, thyroxine, insulin, proinsulin, relaxin, prorelaxin, glycoprotein, follicle stimulating hormone (FSH), thyroid stimulating hormone (TSH), luteinizing hormone (LH), hepatocyte growth factor, fibroblast growth factor, prolactin, placental lactogen, factor-α, tumor growth-β, Mullerian inhibiting substance, gonadotropin-associated peptide mouse, inhibin, activing, vascular endothelial growth factor, integrin, thrombopoietin (TPO), EGF-β growth, platelet growth factor, transforming growth factors (TGF)-α and TGF-β, insulin-like factor-I and -II growth, Erythropoietin (EPO), osteoinductive factors, interferon-α, interferon-β, interferon-γ, macrophage-CSF (M-CSF), CSF granulocyte-macrophage (GM-CSF), granulocyte-CSF (G-CSF) interleukin (IL)-1, IL-1a, IL-2, IL-3, IL-4, IL-5, IL-6, IL-7, IL-8, IL-9, IL-11, IL-12; tumor necrosis factor (TNF)-α, TNF-β, Leukemia Inhibitory Factor (LIF) and kit ligand (KL).

The term "pharmaceutical small molecules" as used herein refers to molecules with a low molecular weight, preferably bellow 8,000 Daltons which can affect other molecules in the surface of the cells such as proteins and lipids leading to a response of the cells, normally cell death. Several databases of small molecules are available such as ChEML (https://www.ebi.ac.uk/chemb), DrugBank (https://go.drugbank.com) and PubChem (https://pubchem.ncbi.nlm.nih.gov) amongst others.

In a second step, the method for determining the effect of a molecule in the cell culture comprises maintaining the support under adequate conditions to allow the functionalizing molecule to exert its effect on the cells of the cell population. Said step further comprises maintaining the support under conditions in which the cells are maintained in a viable status so that any response to the functionalizing molecule is preserved. Typically, this step requires culturing the cells in the adequate conditions to detect the effect of the molecule of interest in the cell culture. This refers to the process of maintaining the cell population viability by supplying the cells with adequate culture media as well as support media additives and placing the cell population under the correct temperature, humidity, light and agitation conditions for the cells to remain viable. Examples of culture media and support media additives are, without limitation, Dulbecco's modified eagle's medium (DMEM), modified eagle's medium (MEM), eagle's basal medium (BME), Roosevelt Park Memorial Institute medium (RPMI), F12 medium, phosphate buffered saline (PBS), L-glutamine, L-alanyl-Lglutamate, non-essential amino acids (NEAA), fetal bovine serum (FBS), bovine calf serum (BCS), horse serum (HS), bovine serum albumin (BSA), human serum albumin (HSA), sodium bicarbonate, sodium carbonate, sodium pyruvate, lipoic acid, ascorbic acid, vitamin B12, nucleosides, cholesterol, oxygenating factors (perfluorocarbons (PFCs), sodium percarbonate, calcium peroxide, magnesium peroxide, hydrogen peroxide), apo- transferrin, insulin, reducing factors (glutathione), Wharton's jelly, and transferrin.

The correct conditions for each cell population is part of the general knowledge of the skilled person in the field.

In a third step, the method for determining the effect of the at least one first functionalizing molecule and/or the at least one second functionalizing molecule on a cell population comprises detecting the response of the cells within the cell population to said functionalizing molecule.

The expression "Detecting the response of the cells within the cell population to said molecule of interest" as used herein refers to the process of identifying at least one parameter that changes in relation to a cell population which is not in contact with the molecule of interest. Said change may be an increase or decrease in the value of said parameter. Said parameter may be a physical parameter of cells, such as cell number, cell proliferation, cell spreading, cell adhesion, cell migration, cell viability, cell secretion, cell growth, cell differentiation, cell morphology, cell apoptosis or any combination thereof. The measurement of cell number is routine experimentation practice and can be accomplished by counting the number of cells in brightfield images acquired just after cell deposition into the support and after one or more determined set times of interest. Cell proliferation can be easily measured by methods and techniques described elsewhere (Chung et al., 2017, Cytometry A. Jul;91(7):704-712; Präbst et al., 2017, Methods Mol Biol.1601:1-17; Romar et al., 2016, J Invest Dermatol. 2016 Jan;136(1):e1-e7). Cell spreading measurement methods are also described elsewhere (Ersoy et al., 2008, ed Image Comput Comput Assist Interv. 11(Pt 1):376-83) as are methods to measure cell proliferation, cell adhesion, cell spreading and cell apoptosis (Minor et al.,Comb Chem High Throughput Screen 2008 Aug;11(7):573-80). Techniques to measure cell growth, proliferation and apoptosis are also common knowledge of the skilled person in the field (Butler et al., 2014, Methods Mol Biol. 1104:169-92). Cell migration can be determined by time lapse microscopy wherein a sequential series of images of the cell population is acquired allowing the movement of cells to be determined and their instantaneous displacement as well as their overall displacement measured. Cell differentiation measurements will depend on the type of differentiation process occurring. Despite this the skilled person in the field will be aware of the best method to determine the differentiation level. Most methods rely on the detection of specific markers which indicate differentiation of a stem cell into a specific cell type or lineage. Just detection can be accomplished by labelling said markers with antibodies or with labels through genetic engineering, wherein said labels can in turn be detected by other methods, such as fluorescence microscopy, western blot, enzymatic assays, etc. Cell morphology and cell secretion can be determined and measured by techniques describe in the literature (Lamers et al., 2010, ur Cell Mater. Nov 9;20:329-43; Torres et al., 2014, Anal. Chem. 86, 23, 11562-11569).

Furthermore, the cell response may be detected by the appearance, disappearance, reduction or increase of a label presented in said cells, wherein said label may be detected in the cell cytosol, surface or in the culture medium. Said label may be a natural label, such as cytokines, or artificial labels such as fluorescent proteins. In another preferred embodiment of the effect method of the invention the response of the cells detected in step (iii) is selected from a group consisting of: cell number, cell proliferation, cell spreading, cell attachment, cell adhesion, cell migration, cell viability, cell secretion, cell growth, cell differentiation, cell morphology, cell death, or any combination thereof.

### Method for determining the capacity of a cell population to secrete a substance of interest

Another aspect of the present invention relates to a method for determining if a cell population secretes to the medium a substance of interest, from here onwards the secretion method of the invention, which comprises the steps of:
(i) Providing the support of the invention wherein said support contains a cell population wherein the cells of the population are attached to the support and wherein the at least one first functionalizing molecule and/or the at least one second functionalizing molecule comprises a molecule which is capable of interacting with the substance of interest, said interaction resulting in a detectable signal;
(ii) Culturing the cells in the adequate conditions to allow binding of the substances of interest secreted to the medium to the at least one first functionalizing molecule and/or the at least one second functionalizing molecule; and
(iii) Detecting the binding of the substance of interest to the at least one first functionalizing molecule and/or the at least one second functionalizing molecule by detecting the signal resulting from said interaction.

All the previous defined terms and expressions are equally valid for the present aspect and its embodiments.

In a first step, the method for determining the capacity of a cell population to secrete a substance of interest comprises providing a support according to the invention wherein said support contains the cell population wherein the cells of the population are attached to the support by adhesion to the compound promoting cell adhesion and wherein the functionalizing molecule comprises a molecule which is capable of interacting with the substance of interest, said interaction resulting in a detectable signal.

It will be understood that the method of the invention is useful not only for the detection of substances which are secreted by the cells and released into the surrounding medium but also for the detection of cellular components which are exposed to the outside of the cell but still associated with the cell such as, for instance, a membrane protein which, upon expression, is targeted to the membrane wherein the luminal domain is exposed to the outside of the cell, becoming available for binding to a specific ligand which is coupled to the microbead as functionalizing molecule.

In another preferred embodiment of the secretion method of the invention the cell population in step (i) is an animal cell population, wherein the animal cells are mammalian cells, preferably selected from a group consisting of: BSC-1 cells, LLC-MK cells, CV-1 cells, CHO cells, COS cells, murine cells, human cells, HeLa cells, 293 cells, VERO cells, MDBK cells, MDCK cells, MDOK cells, CRFK cells, RAF cells, TCMK cells, LLC-PK cells, PK15 cells, WI-38 cells, MRC-5 cells, T-FLY cells, BHK cells, SP2/0 cells, NSO. perC6 (human retina cells) or derivatives thereof.

In another preferred embodiment of the secretion method of the invention the cell population is step (i) is an insect cell population, wherein the insect cells are selected from a group consisting of: Sf21, Sf9, and the BTI-TN-5B1-4 (or High Five) cells, or any combination thereof.

In another preferred embodiment of the secretion method of the invention the cell population is step (i) is a stem cell population, preferably mesenchymal stem cells (MSC), wherein the MSC cells are selected from a group consisting of: bone-marrow MSCs, Adipose MSCs, Umbilical Cord MSCs, ES Cell Derived MSCs and hair follicle derived MSCs, preferably hair follicle derived MSCs.

In a preferred embodiment of the secretion method of the invention in step (i) the cells of the population are attached to the support through a compound which promotes cell adhesion, wherein said compound is selected from a group consisting of: an extracellular matrix molecule selected from the group which consists of: collagen type I, collagen type II, collagen type IV, elastin, fibronectin, vitronectin, laminin or any combination thereof, preferably fibronectin.

The term "substance of interest" as used herein refers to any substance, molecule or protein which the cell population secretes or displays to the culture medium and which is susceptible of being detected by the molecule functionalized to the microbeads. Examples of a substance of interest are biological nanoparticles such as extracellular vesicles or exosomes, cytokines, ions, heavy metal ions, carbohydrates, gases, reactive species, pH and any combination thereof. In a preferred embodiment of the secretion method of the invention the substance of interest of step (i) is selected from a group consisting of: exosomes, extracellular vesicles, lipoproteins, ferritin, viruses, human growth hormone, N-methionyl human growth hormone, bovine growth hormone, parathyroid hormone, thyroxine, insulin, proinsulin, relaxin, prorelaxin, glycoprotein, follicle stimulating hormone (FSH), thyroid stimulating hormone (TSH), luteinizing hormone (LH), hepatocyte growth factor, fibroblast growth factor, prolactin, placental lactogen, factor-α, tumor growth-β, Mullerian inhibiting substance, gonadotropin-associated peptide mouse, inhibin, activing, vascular endothelial growth factor, integrin, thrombopoietin (TPO), EGF-β growth, platelet growth factor, transforming growth factors (TGF)-α and TGF-β, insulin-like factor-I and -II growth, Erythropoietin (EPO), osteoinductive factors, interferon-α, interferon-β, interferon-γ, macrophage-CSF (M-CSF), CSF granulocyte-macrophage (GM-CSF), granulocyte-CSF (G-CSF) interleukin (IL)-1, IL-1a, IL-2, IL-3, IL-4, IL-5, IL-6, IL-7, IL-8, IL-9, IL-11, IL-12; tumor necrosis factor (TNF)-α, TNF-β, Leukemia Inhibitory Factor (LIF), kit ligand (KL), glucose, lactate, oxygen (O₂), calcium (Ca²⁺), carbon dioxide (CO₂), Hydrogen peroxide (H₂O₂), sodium (Na⁺), potassium (K⁺), cadmium (Cd²⁺), cobalt (Co²⁺), palladium (Pb²⁺), copper (Cu²⁺), silver (Ag⁺) , magnesium (Mg²⁺), manganese (Mn²⁺), nickel (Ni²⁺), zinc (Zn²⁺), chromium (Cr³⁺), iron (Fe³⁺), aluminum (Al³⁺), mercury (Hg²⁺) and any combination thereof.

The expression "the at least one first functionalizing molecule and/or the at least one second functionalizing molecule comprises a molecule which is capable of interacting with the substance of interest" refers to the process of the at least one first functionalizing molecule and/or the at least one second functionalizing molecule interacting by binding, processing or reacting with the substance or interest.

In a second step, the method for determining the capacity of a cell population to secrete a substance of interest comprises culturing the cells in the adequate conditions to allow binding of the substances of interest secreted to the medium to the functionalizing molecule.

The expression "Culturing the cells in the adequate conditions to allow binding of the substances of interest secreted to the medium to the functionalizing molecule" refers to the process of maintaining the cell population viability by supplying the cells with adequate culture media as well as support media additives and placing the cell population under the correct temperature, humidity, light and agitation conditions for the cells to remain viable. Examples of culture media and support media additives have been mentioned previously and are equally valid for the current aspect. By maintaining the cell population viability, the cells will continue to produce and secrete the substance of interest which will then bind to the functionalizing molecule.

In a preferred embodiment of the secretion method of the invention the at least one first functionalizing molecule and/or the at least one second functionalizing molecule is selected form a group consisting of: antibody, any of the functionalizing molecules of Table 1 and any combination thereof.

**Table 1: List of functionalizing molecules for the secretion method of the invention together with the substance which can generates a detectable signal.**

| **Functionalizing molecule** | **Substance of interest detected** | **References** |
|---|---|---|
| Glucose-Responsive Monomer | Glucose | Shibata et al., 2010, PNAS 107 (42) 17894-17898 |
| Glucose oxidase | Glucose | Kim et al., 2016, Polym. Chem., 7, 1907-1912 |
| Lactate oxidase | Lactate | Zhang et al., 2017, ACS Appl. Mater. Interfaces 9, 44, 38153-38158 |
| Extracellular Oxygen Consumption Reagent | O₂ | Zhu et al., 2019, Theranostics May 18;9(11):3293- 3307 |
| Pt-porphyrin | O₂ | Saito et al., 2007,Journal of Porphyrins and Phthalocyanines, 11, 03, pp. 160-164 |
| 4,4-difluoro-4-bora-3a,4a-diaza-s-indacene(BODIPY)-based fluorophore and a Ca2+ recognition group | Ca²⁺ | Sui et al., 2016, Chemistry 18, 22(30): 10351-4 |
| Solvatochromic Probe | CO₂ | Ali et al., 2011, Anal. Chem., 83, 8, 2846-2851 |
| Dicationic mesoBis(benzimidazoli um) Calix[4]pyrrole | CO₂ | Mulugeta et al., 2017, Chem 3, 1008-1020 |
| H₂O₂-selective probes | H₂O₂ | Grisham, 2013, Comp Biochem Physiol A Mol Integr Physiol Aug;165(4): 429-38. |
| Sensing Indicators | Na⁺ and K⁺ | https://www.thermofisher.com/es/es/home/refer ences/molecular-probes-the-handbook/indicators-for-na-k-cl-and-miscellaneous-ions/fluorescent-na-and-k-indicators.html (accessed on the 30/07/2021) |
| Aggregation-Induced Emission | Heavy metals | Neupane et al., 2016, Anal. Chem., 88, 6, 3333-3340 |
| FRET | pH | Liu *et al.,* 2020, Front. Bioeng. Biotechnol., Nov 9;8:595497 |
| Fluorescence Probes | pH | Chen, 2021, Anal Biochem, Jan 1;612:113900 |

As used herein, the term "antibody" refers to monoclonal antibodies (mAbs), multispecific antibodies, human antibodies, humanized antibodies, synthetic antibodies, chimeric antibodies, polyclonal antibodies, camel antibodies, single chain FVS antibodies, single-chain antibodies, immunologically active antibody fragments (e.g., antibody fragments capable of binding epitopes, e.g., Fab fragments, Fab' fragments, F(AB')2 fragments, Fv fragments, fragments containing a VL or VH domain or complementarity determining regions (CDRs) immunospecifically bind antigen, etc.), bi- or poly-functional antibodies, disulfide-linked bispecific FVS (sdFv), intrabody and diabodies, and any of the above epitope binding fragments. In particular, the term "antibody" is intended to include immunologically active fragments of immunoglobulin molecules and immunoglobulin molecules, i.e., molecules containing an antigen binding site. Immunoglobulin molecules can be of any type (e.g., IgG, IgE, IgM, IgD, IgA, and IgY), class (e.g., IgGbIgG2, IgG3, IgG4, IgAi, and IgA2) or subclass.

In a third step, the method for determining if a cell population secretes to the medium a substance of interest which comprises detecting the binding of the substance of interest to the at least one first functionalizing molecule and/or the at least one second functionalizing molecule by detecting the signal resulting from said interaction.

The expression "Detecting the binding of the substance of interest to the at least one first functionalizing molecule and/or the at least one second functionalizing molecule by detecting the signal resulting from said interaction" as used herein refers to the process of identifying the binding of the substance of interest to the at least one first functionalizing molecule and/or the at least one second functionalizing molecule. Said detection may be a direct detection of the substance of interest through the use of molecules which bind to it, such as aptamers, such as structure switching signaling aptamers (SSSA) or antibodies, such as primary antibodies anti-substance of interest labelled or a conjugation of primary antibodies anti-substance of interest with secondary antibody anti primary antibody, wherein the secondary antibody is labeled with a detectable marker. Detection may be performed trough optical microscopy or fluorescence microscopy.

### Method for determining the effect of an effector molecule on a cell population and for determining if the cell population secretes to the medium a substance of interest

The authors of the present invention have found that, by combining in the same support gel and/or microbeads functionalizing molecules with a effector function on the cell population within the support and gel and/or microbeads containing functionalizing molecules capable of interacting with a substance of interest, the support of the present invention allows the simultaneous detection of an effect of a functionalizing molecule on a cell population and the production of a cell of interest by said cell population.

Another aspect of the present invention relates to a method for determining the effect of an effector molecule on a cell population and for determining if the cell population secretes to the medium a substance of interest, from here on the dual method of the invention, which comprises the steps of:
(i) Providing the support of the invention wherein said support contains the cell population, wherein the cells of the population are attached to the support and wherein:
   - the gel comprises a first type of at least one first functionalizing molecule which is the effector molecule and a second type of at least one first functionalizing molecule which is capable of interacting with the substance of interest;
   - the gel comprises a first type of at least one first functionalizing molecule which is the effector molecule and the microbeads comprise a second type of at least one second functionalizing molecule which is capable of interacting with the substance of interest;
   - the microbeads comprise a first type of at least one second functionalizing molecule which is the effector molecule and the gel comprises a second type of at least one first functionalizing molecule which is capable of interacting with the substance of interest; or
   - the first and second type of microbeads contain, respectively, a first type of second functionalizing molecule which is the effector molecule and a second type of second functionalizing molecule which is capable of interacting with the substance of interest,
   and wherein the interaction of the first functionalizing molecule or of the second functionalizing molecule with the substance of interest results in a detectable signal;
(ii) Culturing the cells under conditions adequate to allow the effector molecule to exert its effect on the cells of the cell population and the binding of the substances of interest secreted to the medium to the detector molecule; and
(iii) Detecting the response of the cells within the cell population to said effector molecule and detecting the binding of the substance of interest to the detector molecule by detecting the signal resulting from said interaction.

All the previous defined terms and expressions are equally valid for the present aspect and its embodiments.

In another embodiment of the dual method of the invention the effector molecule is selected from a group consisting of growth factors, differentiation factors, cell adhesion molecules or proteins, pharmaceutical small molecules and any combination thereof.

In yet another embodiment of the dual method of the invention, the growth factor is selected from the group consisting of: vascular endothelial growth factor (VEGF), collagen, bone morphogenic factor-β, epidermal cell growth factor (EGF), platelet derived growth factor (PDGF), nerve growth factor (NGF), fibroblast growth factor (FGF), insulin-like growth factor (IGF), transforming growth factor (TGF) and any combination thereof.

In an additional embodiment of the dual method of the invention the substance of interest of step (i) is selected from a group consisting of: exosomes, extracellular vesicles, lipoproteins, ferritin, viruses, human growth hormone, N-methionyl human growth hormone, bovine growth hormone, parathyroid hormone, thyroxine, insulin, proinsulin, relaxin, prorelaxin, glycoprotein, follicle stimulating hormone (FSH), thyroid stimulating hormone (TSH), luteinizing hormone (LH), hepatocyte growth factor, fibroblast growth factor, prolactin, placental lactogen, factor-α, tumor growth-β, Mullerian inhibiting substance, gonadotropin-associated peptide mouse, inhibin, activing, vascular endothelial growth factor, integrin, thrombopoietin (TPO), EGF-β growth, platelet growth factor, transforming growth factor (TGF)-α, TGF-β, insulin-like factor-I growth, insulin-like factor-II growth, Erythropoietin (EPO), osteoinductive factor, interferon-α, interferon-β, interferon---γ, macrophage-CSF (M-CSF), CSF granulocyte-macrophage (GM-CSF), granulocyte-CSF (G-CSF) interleukin (IL)-1, IL-1a, IL-2, IL-3, IL-4, IL-5, IL-6, IL-7, IL-8, IL-9, IL-11, IL-12; tumor necrosis factor(TNF)-α, TNF-β, leukemia inhibitory factor (LIF), kit ligand (KL), glucose, lactate, oxygen (O₂), calcium (Ca²⁺), carbon dioxide (CO₂), Hydrogen peroxide (H₂O₂), sodium (Na⁺), potassium (K⁺), cadmium (Cd²⁺), cobalt (Co²⁺), palladium (Pb²⁺), copper (Cu²⁺), silver (Ag⁺) , magnesium (Mg²⁺), manganese (Mn²⁺), nickel (Ni²⁺), zinc (Zn²⁺), chromium (Cr³⁺), iron (Fe³⁺), aluminum (Al³⁺), mercury (Hg²⁺) and any combination thereof.

On more preferred embodiment of the dual method of the invention the effect of the effector molecule on the cell population is the secretion of the substance of interest.

The present invention relates in addition to the following aspects:
1. A support suitable for culturing cells characterized in that it shows patterned regions, said patterned regions being defined by:
   - a complementary pattern of a uniformly distributed gel attached to the support, wherein the gel comprises at least one first functionalizing molecule, or
   - a complementary pattern comprising two or more complementary patterned regions, wherein at least one complementary pattern region is defined by an uniformly distributed gel attached to the support and at least one complementary pattern region is defined by uniformly distributed microbeads attached to the support, wherein the gel comprise at least one first functionalizing molecule and/or the microbeads are modified with at least one second functionalizing molecule,
   wherein the patterned regions in the support show increased adhesive capacity to cells with respect to the complementary pattern to which the gel or the gel and microbeads are attached.
2. The support according to aspect 1 wherein the patterned regions are defined by:
   - a complementary pattern of an uniformly distributed gel attached to the support and wherein the complementary pattern of the uniformly distributed gel is created by allowing the gel to adhere and solidify onto the support when the support is in contact with a stamp, said stamp having protrusions which define the pattern regions within the support, thereby preventing the gel from attaching to those areas of the support which are in contact with the stamp protrusions,
      or by
   - a complementary pattern comprising two or more complementary pattern regions, wherein at least one complementary pattern region is defined by an uniformly distributed gel attached to the support and at least one complementary patterned region is define by uniformly distributed microbeads attached to the support, wherein the complementary pattern regions are created by allowing the gel and microbeads to adhere and solidify onto the support when the support is in contact with a stamp, said stamp having protrusions which define the patterned regions within the support, thereby preventing the gel and microbeads from attaching to those areas of the support which are in contact with the stamp protrusions.
3. The support according to any one of aspects 1 or 2 wherein complementary pattern of a uniformly distributed gel attached to the support or the complementary pattern region of a uniformly distributed gel attached to the support has a height of between 1 µm and 200 µm.
4. The support according to any of aspects 1 to 3 wherein the regions in the support onto which the gel or gel and microbeads are attached are further coated with a compound or composition which substantially reduces cell adhesion.
5. The support according to any of aspects 2 to 4 wherein the patterned regions are created using vacuum-driven soft lithography.
6. The support according to any one of aspects 1 to 5 wherein the gel is a hydrogel or an ionogel.
7. The support according to aspect 6 wherein the gel is a hydrogel comprising a polymer selected from a group consisting of: alginic acid, agarose, hyaluronic acid, polyethylene glycol, gelatin, collagen, fibrin, chitosan, fibrin, fibrinogen, fibronectin, laminin, elastin, and combinations thereof.
8. The support according to aspect 7 wherein the hydrogel polymer is gelatin, preferably gelatin methacrylate.
9. The support according to aspect 6 wherein the gel is an ionogel comprising an ionic liquid selected from the group consisting of: 1-ethyl-3-methylimidazolium ethyl sulphate, trihexyltetradecylphosphonium dicyanamide, choline acetate, 1-butyl-2,3-dimethylimidazolium, 1-butyl-3-methylimidazolium, 1-butyl-1-methylpyrrolidinium, 1-butyl-1-methylpiperidinium, and methyltrioctylammonium.
10. The support according to any one of aspects 1 to 9 wherein the diameter of the microbeads is between 50 nm and 25000 nm.
11. The support according to aspect 1 to 11 wherein the microbeads are selected from a group consisting of: glass microbeads, polystyrene microbeads, poly(methyl methacrylate) microbeads, silica microbeads, zirconia microbeads, titanium microbeads, gold microbeads, polyethylenepolylactic acid (PLA) microbeads, poly-L-lactic acid (PLLA) microbeads, poly glycolic acid (PGA) microbeads, poly lactic-co-glycolic acid (PLGA) microbeads or poly-caprolactone (PCL) microbeads, preferably polystyrene microbeads.
12. The support according to any one of aspects 1 to 11 wherein the second functionalizing molecule is attached to the microbeads through a coupling molecular pair, wherein one member of the coupling molecular pair is bound to the microbeads and the other member of the coupling molecular pair is bound to the molecule of interest.
13. The support according to aspect 12 wherein the coupling molecule pair is the streptavidin biotin molecular pair.
14. The support according to any of aspects 1 to 13 containing at least two types of microbeads wherein each type of microbead is modified with at least one second functionalizing molecule, wherein the functionalizing molecules are identical or different between the different types of microbeads.
15. The support according to any of aspects 1 to 14 wherein the gel comprises at least two different types of first functionalizing molecules.
16. The support according to any of aspects 1 to 15 wherein the gel is polymerized in the presence of the at least one first functionalizing molecule.
17. The support according to any of aspects 1 to 15 wherein the at least one first functionalizing molecule is added to the gel after the gel polymerization.
18. The support according to any of aspects 1 to 17 wherein the gel comprises at least two first functionalizing molecules, wherein at least one first functionalizing molecule is present when the gel is polymerized and at least another first functionalizing molecule is added to the gel after the gel polymerization.
19. The support according to any of aspects 1 to 18 wherein the gel is polymerized from a gel solution through UV irradiation.
20. The support according to any of aspects 1 to 19 wherein the patterned regions in the support are coated with a compound or composition that promotes cell adhesion.
21. The support according to aspect 20 wherein the coating of the support with a compound or composition that promotes cell adhesion is carried out by coating the protrusions of the stamp with said compound or composition that promotes cell adhesion and contacting the support with the stamp thereby allowing the transfer of the compound or composition from the stamp onto the support with a pattern that matches the pattern of protrusions of the stamp.
22. The support according to aspects 20 or 21 wherein the compound that promotes cell adhesion is an extracellular matrix molecule, preferably fibronectin.
23. The support according to any of aspects 1 to 22 where in the support is made from a material selected from a group consisting of: glass, borosilicate glass, quartz glass, polystyrene, polymethylmethacrylate (PMMA), polycarbonate, polyethylene and cyclic olefin copolymers.
24. The support according to any of aspects 1 to 23 wherein the patterned regions have a geometrical form, preferably a circular form with a diameter of between 10 µm to 200 µm.
25. The support according to any of aspects 1 to 24 wherein the support further contains viable cells which are attached to the support or to molecules within the support which promote cell adhesion.
26. A method for producing the support according to any of aspects 1 to 25 selecting from:
   (a) a method comprising the steps of:
      (i) Providing a support;
      (ii) Contacting the support with a stamp, said stamp having protrusions which define the desired pattern in the support, wherein the contact of the support with the stamp forms bottomless channels between the support and the stamp,
      (iii) contacting the support along the bottomless channels with a gel solution and allowing the gel solution to distribute uniformly in those regions in the support which are not protected by the protrusions of the stamp, and
      (iv) polymerizing the gel in order for the gel to adhere to the support as to obtain a uniform attachment onto those regions in the support which are not protected by the protrusions of the stamp obtaining a pattern defined by the gel which is complementary to the desired pattern, wherein the gel solution comprises at least one first functionalizing molecule or at least one first functionalizing molecule is added to the gel after the gel is polymerized;
      or
   (b) a method comprising the steps of:
      (i) Providing a support;
      (ii) Contacting the support with a stamp, said stamp having protrusions which define the desired pattern in the support, wherein the contact of the support with the stamp forms bottomless channels between the support and the stamp,
      (iii) contacting the support along the bottomless channels with a gel solution and a microbead suspension wherein the microbeads are modified with at least one second functionalizing molecule and allowing the microbeads to adhere to the support as to obtain a uniform deposition in and attachment of the microbeads onto those regions in the support which are not protected by the protrusions of the stamp nor occupied by the gel solution, and
      (iv) and polymerizing the gel in order for the gel to adhere to the support as to obtain a uniform attachment onto those regions in the support which are not protected by the protrusions of the stamp nor occupied by the microbeads, obtaining a pattern defined by the gel and microbeads which is complementary to the desired pattern,

   wherein the gel comprises at least one first functionalizing molecule and/or the microbeads are modified with at least one second functionalizing molecule and
   wherein the gel solution comprises at least one first functionalizing molecule or at least one first functionalizing molecule is added to the gel after the gel is polymerized.
27. The method according to aspect 26 wherein the protrusions of the stamp in step (ii) of method (a) and (b) are coated with a compound or composition that promotes cell adhesion thereby allowing the transfer of the compound or composition from the stamp onto the support with a pattern that matches the pattern of protrusions of the stamp.
28. The method according to any of aspects 26 or 27 wherein the support of step (i) of method (a) and (b) is made from a material selected from a group consisting of: glass, borosilicate glass, quartz glass, polystyrene, polymethylmethacrylate (PMMA), polycarbonate, polyethylene, cyclic olefin copolymers preferably glass.
29. The method according to any of aspects 26 to 28 wherein the stamp is a PDMS stamp.
30. The method according to aspects 26 or 29 wherein step (ii) of method (a) and (b) is carried out by vacuum-driven soft lithography.
31. The method according to any of aspects 26 to 30 wherein the regions in the support onto which the microbeads attaches in step (iii) of method (b) and onto which the gel attaches in step (iv) of method (a) and (b) are further coated with a compound or composition which substantially reduces cell adhesion.
32. The method according to any of aspects 26 to 31 wherein the gel is a hydrogel or an ionogel.
33. The method according to aspect 32 wherein the gel is a hydrogel and comprises a polymer selected from a group consisting of: alginic acid, agarose, hyaluronic acid, polyethylene glycol, gelatin, collagen, fibrin, chitosan, fibrin, fibrinogen, fibronectin, laminin, elastin, and combinations thereof.
34. The method according to aspect 33 wherein the hydrogel polymer is gelatin.
35. The method according to aspect 34 wherein the gel is an ionogel comprising an ionic liquid selected from the group consisting of: 1-ethyl-3-methylimidazolium ethyl sulphate, trihexyltetradecylphosphonium dicyanamide, choline acetate, 1-butyl-2,3-dimethylimidazolium, 1-butyl-3-methylimidazolium, 1-butyl-1-methylpyrrolidinium, 1-butyl-1-methylpiperidinium, and methyltrioctylammonium.
36. The method according to any of aspects 26 to 35 wherein the complementary pattern defined by the gel has a height of between 31 µm and 200 µm.
37. The method according to any of aspects 26 to 36 wherein the microbeads from step (iii) of method (b) are selected from a group consisting of: glass microbeads, polystyrene microbeads, polyacrylamide microbeads, poly(methyl methacrylate) microbeads, silica microbeads, zirconia microbeads, titanium microbeads, gold microbeads, polyethylenepolylactic acid (PLA) microbeads, poly-L-lactic acid (PLLA) microbeads, poly glycolic acid (PGA) microbeads, poly lactic-co-glycolic acid (PLGA) microbeads and poly-caprolactone (PCL) microbeads, preferably polystyrene microbeads.
38. The method according to any of aspects 26 to 37 wherein the microbeads of step (iii) from method (b) have a diameter between 50 nm and 25000 nm.
39. The method according to any of aspects 26 to 38 wherein in step (iii) of method (a) and (b) the chamber formed between the stamp and the support is provided with at least one operable inlet and optionally at least one operable outlet.
40. The method according to aspect 39 wherein step (iii) of method (a) is carried out by injecting a gel solution at a temperature wherein the gel is liquefied through the at least one inlet, maintaining the gel solution within the channels for a sufficient time so as to obtain a uniform distribution of the gel on the support before proceeding with step (iv).
41. The method according to aspect 39 wherein step (iii) of method (b) is carried out by injecting a gel solution at a temperature wherein the gel solution is liquefied through the at least one inlet and by injecting a suspension of microbeads through the at least one inlet, maintaining the gel solution within the channels for a sufficient time so as to obtain a uniform distribution of the gel and a uniform deposition of the microbeads on the support before proceeding with step (iv).
42. The method according to any of aspects 26 to 41 wherein in step (iii) and step (iv) of method (a) and (b) the gel solution or the gel solution and the microbead suspension further comprises a compound or composition which substantially reduces cell adhesion or a compound of composition of interest.
43. The method according to any of aspects 26 to 42 wherein step (iv) of method (a) and (b) is carried out by UV irradiation with a wavelength of between 350 nm and 400 nm.
44. The method according to aspect 43 wherein the UV irradiation is carried out in cycles, each cycle consisting of 5 seconds (s) of irradiation (ON) followed by 30 seconds without irradiation (OFF), for between 5 to 70 cycles.
45. The method according to any of aspects 26 to 45 wherein step (iii) and step (iv) of method (a) and (b) is carried out while pressure is being applied to bring into contact the slab and the support.
46. The method according to aspect 45 wherein the pressure is achieved by creating vacuum in the chamber formed between the support and the slab.
47. The method according to any of aspects 26 to 46 further comprising contacting the support with a cell population under conditions adequate for the binding of the cells to the support through interactions between the cells and the compound in the support that promotes cell adhesion and the formation of patterned cell clusters.
48. The method according to aspect 47 wherein the cell population is placed inside a well-like structure wherein said well-like structure is formed by a base and vertical walls wherein the base is formed by the support layer comprising the predetermined patterns base.
49. The method according to aspect 48 wherein the well-like structure vertical walls are made from a material selected from a group consisting of: polydimethylsiloxane (PDMS), glass, poly(methyl methacrylate) (PMMA), pressure sensitive adhesive sheets (PSA), cyclic olefin copolymer (COC), cyclic olefin polymers (COP), polyethylene terephthalate (PET), BioMed Clear Resin (Formlabs), polyether ether ketone (PEEK), polystyrene (PS), polypropylene (PP), polyethylene (PE), polycarbonate or any combination thereof.
50. A method for determining the effect of a molecule on a cell population which comprises the steps of:
   (iv) Providing a support according to any of the aspects 1 to 25 wherein said support contains the cell population wherein the cells of the population are attached to the support and wherein the molecule, the effect of which on the cell population is to be determined is the at least one first functionalizing molecule and/or the at least one second functionalizing molecule;
   (v) Maintaining the support under adequate conditions to allow the at least one first functionalizing molecule and/or the at least one second functionalizing molecule to exert its effect on the cells of the cell population; and
   (vi) Detecting the response of the cells within the cell population to said first functionalizing molecule and/or the at least one second functionalizing molecule,
   wherein the detection of a response of the cells to the at least one first functionalizing molecule and/or the at least one second functionalizing molecule is indicative that the at least one first functionalizing molecule and/or the at least one second functionalizing molecule exerts an effect on the cells of the cell population.
51. The method according to aspect 50 wherein the at least one first functionalizing molecule and/or the at least one second functionalizing molecule is selected from a group consisting of growth factors, differentiation factors, cell adhesion molecules or proteins, pharmaceutical small molecules and any combination thereof.
52. The method according to aspect 51 wherein the growth factor is selected from the group consisting of: vascular endothelial growth factor (VEGF), collagen, bone morphogenic factor-β, epidermal cell growth factor (EGF), platelet derived growth factor (PDGF), nerve growth factor (NGF), fibroblast growth factor (FGF), insulin-like growth factor (IGF), transforming growth factor (TGF) and any combination thereof.
53. A method for determining if a cell population secretes to the medium a substance of interest which comprises the steps of:
   (iv) Providing a support according to any of the aspects 1 to 25 wherein said support contains a cell population wherein the cells of the population are attached to the support and wherein the at least one first functionalizing molecule and/or the at least one second functionalizing molecule comprises a molecule which is capable of interacting with the substance of interest, said interaction resulting in a detectable signal;
   (v) Culturing the cells in the adequate conditions to allow binding of the substances of interest secreted to the medium to the at least one first functionalizing molecule and/or the at least one second functionalizing molecule; and
   (vi) Detecting the binding of the substance of interest to the at least one first functionalizing molecule and/or the at least one second functionalizing molecule by detecting the signal resulting from said interaction.
54. The method according to aspect 53 wherein the substance of interest of step (i) is selected from a group consisting of: exosomes, extracellular vesicles, lipoproteins, ferritin, viruses, human growth hormone, N-methionyl human growth hormone, bovine growth hormone, parathyroid hormone, thyroxine, insulin, proinsulin, relaxin, prorelaxin, glycoprotein, follicle stimulating hormone (FSH), thyroid stimulating hormone (TSH), luteinizing hormone (LH), hepatocyte growth factor, fibroblast growth factor, prolactin, placental lactogen, factor-α, tumor growth-β, Mullerian inhibiting substance, gonadotropin-associated peptide mouse, inhibin, activing, vascular endothelial growth factor, integrin, thrombopoietin (TPO), EGF-β growth, platelet growth factor, transforming growth factor (TGF)-α, TGF-β, insulin-like factor-I growth, insulin-like factor-II growth, Erythropoietin (EPO), osteoinductive factor, interferon-α, interferon-β, interferon---γ, macrophage-CSF (M-CSF), CSF granulocyte-macrophage (GM-CSF), granulocyte-CSF (G-CSF) interleukin (IL)-1, IL-1a, IL-2, IL-3, IL-4, IL-5, IL-6, IL-7, IL-8, IL-9, IL-11, IL-12; tumor necrosis factor(TNF)-α, TNF-β, leukemia inhibitory factor (LIF), kit ligand (KL), glucose, lactate, oxygen (O₂), calcium (Ca²⁺), carbon dioxide (CO₂), Hydrogen peroxide (H₂O₂), sodium (Na⁺), potassium (K⁺), cadmium (Cd²⁺), cobalt (Co²⁺), palladium (Pb²⁺), copper (Cu²⁺), silver (Ag⁺) , magnesium (Mg²⁺), manganese (Mn²⁺), nickel (Ni²⁺), zinc (Zn²⁺), chromium (Cr³⁺), iron (Fe³⁺), aluminum (Al³⁺), mercury (Hg²⁺) and any combination thereof.
55. A method for determining the effect of an effector molecule on a cell population and for determining if the cell population secretes to the medium a substance of interest which comprises the steps of:
   (i) Providing a support according to any of aspects 1 to 25 wherein said support contains the cell population, wherein the cells of the population are attached to the support and wherein:
      - the gel comprises a first type of at least one first functionalizing molecule which is the effector molecule and a second type of at least one first functionalizing molecule which is capable of interacting with the substance of interest;
      - the gel comprises a first type of at least one first functionalizing molecule which is the effector molecule and the microbeads comprise a second type of at least one second functionalizing molecule which is capable of interacting with the substance of interest;
      - the microbeads comprise a first type of at least one second functionalizing molecule which is the effector molecule and the gel comprises a second type of at least one first functionalizing molecule which is capable of interacting with the substance of interest; or
      - the first and second type of microbeads contain, respectively, a first type of second functionalizing molecule which is the effector molecule and a second type of second functionalizing molecule which is capable of interacting with the substance of interest,
      and wherein the interaction of the first functionalizing molecule or of the second functionalizing molecule with the substance of interest results in a detectable signal;
   (ii) Culturing the cells under conditions adequate to allow the effector molecule to exert its effect on the cells of the cell population and the binding of the substances of interest secreted to the medium to the detector molecule; and
   (iii) Detecting the response of the cells within the cell population to said effector molecule and detecting the binding of the substance of interest to the detector molecule by detecting the signal resulting from said interaction.
56. The method according to aspect 55 wherein the effector molecule is selected from a group consisting of growth factors, differentiation factors, cell adhesion molecules or proteins, pharmaceutical small molecules and any combination thereof.
57. The method according to aspect 56 wherein the growth factor is selected from the group consisting of: vascular endothelial growth factor (VEGF), collagen, bone morphogenic factor-β, epidermal cell growth factor (EGF), platelet derived growth factor (PDGF), nerve growth factor (NGF), fibroblast growth factor (FGF), insulin-like growth factor (IGF), transforming growth factor (TGF) and any combination thereof.
58. The method according to any of aspects 56 to 57 wherein the substance of interest of step (i) is selected from a group consisting of: exosomes, extracellular vesicles, lipoproteins, ferritin, viruses, human growth hormone, N-methionyl human growth hormone, bovine growth hormone, parathyroid hormone, thyroxine, insulin, proinsulin, relaxin, prorelaxin, glycoprotein, follicle stimulating hormone (FSH), thyroid stimulating hormone (TSH), luteinizing hormone (LH), hepatocyte growth factor, fibroblast growth factor, prolactin, placental lactogen, factor-α, tumor growth-β, Mullerian inhibiting substance, gonadotropin-associated peptide mouse, inhibin, activing, vascular endothelial growth factor, integrin, thrombopoietin (TPO), EGF-β growth, platelet growth factor, transforming growth factor (TGF)-α, TGF-β, insulin-like factor-I growth, insulin-like factor-II growth, Erythropoietin (EPO), osteoinductive factor, interferon-α, interferon-β, interferon---γ, macrophage-CSF (M-CSF), CSF granulocyte-macrophage (GM-CSF), granulocyte-CSF (G-CSF) interleukin (IL)-1, IL-1a, IL-2, IL-3, IL-4, IL-5, IL-6, IL-7, IL-8, IL-9, IL-11, IL-12; tumor necrosis factor(TNF)-α, TNF-β, leukemia inhibitory factor (LIF), kit ligand (KL), glucose, lactate, oxygen (O₂), calcium (Ca²⁺), carbon dioxide (CO₂), Hydrogen peroxide (H₂O₂), sodium (Na⁺), potassium (K⁺), cadmium (Cd²⁺), cobalt (Co²⁺), palladium (Pb²⁺), copper (Cu²⁺), silver (Ag⁺) , magnesium (Mg²⁺), manganese (Mn²⁺), nickel (Ni²⁺), zinc (Zn²⁺), chromium (Cr³⁺), iron (Fe³⁺), aluminum (Al³⁺), mercury (Hg²⁺) and any combination thereof.
59. The method according to any of aspects 55 to 58 wherein the effect of the effector molecule on the cell population is the secretion of the substance of interest.

The invention will be described by way of the following examples which are to be considered as merely illustrative and not limitative of the scope of the invention.

### EXAMPLES

### 1. Materials and Methods

### 1.1 - Materials

Gelatin methacrylate (GelMa) was purchased from Celllnk. N-isopropylacrylamide (NIPAAm) monomer, *N,N'-*methylene-bis(acrylamide) (mBAAm), 2,2-dimethoxy-2-phenylacetophenone (DMPA), 1-ethyl-3-methylimidazolium ethyl sulphate (EMIES), trihexyltetradecylphosphonium dicyanamide (DCA), choline acetate (ChAc), 3-(trimethoxysilyl)propil methacrylate, bovine serum albumin (BSA) were purchased from Sigma Aldrich, Spain. Streptavidin coated polystyrene microbeads (200 nm Ø) were purchased from Immunostep, Spain. Biotinylated FGF-2 was purchase from Deltaclon, Spain. Polymethyl methacrylate (PMMA) Plexiglas 4mm was purchased from Evonik Industries AG, Germany. Pressure Sensitive Adhesive (PSA) ARcare 8939 was purchased from Adhesive Research, Ireland.

Human adult Mesenchymal Stromal Cells were obtained from human follicles (hHF MCSs, p4). HeLa cells were kindly donated by the "Tissue Engineering and Novel Regenerative Strategies Group", CIMA, University of Navarra. VEGF, cell culture t75 flasks, goat anti-mouse polyclonal IgG Alex Fluor 488 and casein were purchased from Fisher Scientific, Spain. Complete Medium 1 (CM1) consisted of Dulbecco's Modified Eagle's Medium (DMEM) (Fisher Scientific Spain) supplemented with 30% Fetal Bovine Serum (FBS) (Fisher Scientific Spain) and 10% Penicillin/Streptomycin (P/S) (Fisher Scientific Spain) while complete Medium 2 (CM2) consisted of DMEM supplemented with 10% FBS and 1% P/S. Serum-free DMEM medium 1 (SFM1) FBS consisted in Dulbecco's Modified Eagle's Medium (DMEM) (Fisher Scientific Spain) with 10% Penicillin/Streptomycin (P/S) (Fisher Scientific Spain) while serum-free DMEM medium 2 (SFM2) consisted in DMEM with 1% P/S. Biotin goat anti-VEGF polyclonal IgG and mouse anti-VEGF monoclonal IgG were purchased from R&D Systems, USA. Paraformaldehyde 4% was purchased from Panreac Quimica, Spain. Milk powder was obtained from Nativa 2, Nestle. 4',6-diamino-2-phenylin (DAPI) dye, phalloidin dye and Live/Dead viability kit were purchased from Thermo Fisher Scientific.

Primary antibody (Primary Ab) solution consisted of mouse monoclonal anti-VEGF IgG (1 µg mL⁻¹), 0.2 % milk powder (w/v), 1 % goat serum (v/v) in PBS. Secondary antibody (Secondary Ab) solution consisted of goat anti-mouse IgG (1 µg mL⁻¹), 0.2 % milk powder (w/v), 1 % goat serum (v/v) in PBS.

### 1.2 - Patterning of gels through Vacuum lithography

The microstructures were fabricated through the Vacuum Lithography (PnVlitho). Three different ionogels and GelMa were employed. The three ionogels were made using different ionic liquids: EMIES (IO-1), DCA (IO-2) and ChAc (IO-3). For the IO-1,452 mg NIPAAm, 30.9 mg mBAAm, 30 mg DMPA and 1 mL of EMIES ionic liquid were mixed. For the IO-2, 452 mg NIPAAm, 30.9 mg mBAAm, 30 mg DMPA and 1.5 mL of DCA were mixed. For the third ionogel, IO-3, 1 g of ChAc was melted at 120 °C for 1 h and afterwards 452 mg NIPAAm, 30.9 mg mBAAm, 30 mg DMPA were added. The three ionogels were kept in stirring at 100 °C until all the solid compounds were dissolved. GelMa gels were worked at 37 °C throughout the whole process.

For the fabrication of the microstructures, the substrate, in this case cover glasses, were chemically functionalized. Glass covers (24 x 60 mm) were oxidized with air plasma (Harrick PDC-32G-2) for 30 min, followed by vapor deposition of 3-(trimethoxysilyl)propyl methacrylate for 2 h under vacuum. Then, PDMS stamps were placed in close contact with the cover glasses. PDMS slabs wave channel-like structure were used (two parallel wave-like channels of 400 µm width × 8200 µm length × 33 µm height separated 100 µm between channels). Afterwards, the resulted pasted PDMS slabs on glass were put under vacuum inside a desiccator for 20 min. The outlets were closed with tape, and 3.5 µL of either one of the three ionogels or GelMa were loaded in the inlets. Both the ionogels and the GelMa were heated at 70 °C and 40 °C respectively for 1 h before loading.

For the polymerization of the ionogels and the GelMa, samples were exposed in cycles to UV light (365 nm) (UV-KUB 2 lamp) for 5 s (460 mJ) every 30s. Different UV light cycles were studied for the ionogels (5, 10, 20, 30, 50 and 70) and GelMa (10 and 20). At end of the respectively cycle, PDMS stamps were removed, and the height of the features of the microstructures generated were measured by optical profilometry with a DektakXT (Bruker) profilometer.

### 1.3 - Co-patterning of gels and hHF-MSCs

To combine the ionogel structures and cells, Printing and Vacuum Lithography (PnV Litho) was carried out, for which PDMS slabs wave channel-like structure were used (two parallel wave-like channels of 400 µm width x 8200 µm length x 33 µm height separated 100 µm between channels). The separation between the channels was wetted with a solution of fibronectin 100 µg mL⁻¹ for 30 min. Afterwards, PDMS slabs were rinsed with distilled water, dried with compressed air and attached to the glass cover previously functionalized. Subsequently, the vacuum lithography protocol was followed (Section 2.2), using IO-3 as the gel for the formation of the patterns. PDMS slabs were left overnight for solvent evaporation at room temperature.

PDMS slabs were removed and the wells were loaded with 1 mL of blocking solution (BSA 5 % (w/v), milk powder 0.1 % (w/v) and casein 1 % (v/v) in PBS) for 2 h. Then, hair Human Follicle derived Mesenchymal Stem Cells (hHF-MSCs) were detached from the flasks and were resuspended in serum free medium (SFM1) at a concentration of 10⁵ cells mL⁻¹. 750 µL of the cell suspension were loaded to the pattern of fibronectin surrounded by ionogel, and were left inside the incubator on constant oscillation in a rocker (Vari-Mix steep angle rocker, Thermo Fisher) for 60 min (n = 3 per experimental condition). Afterwards, the medium was retrieved and the wells were rinsed three times with PBS to wash out any non-attached cell. At the end of the assays, patterned cells were fixed with paraformaldehyde 4 % and stained with DAPI (1:1000 from stock solution) and phalloidin while others were stained with the Live/Dead viability kit. Afterwards they were photographed.

For the adhesion of cells in the form of small cell islands, PDMS slabs channel-like structures (1000 × 5000 × 13 µm, width × length × height) with pillars inside (100 µm diameter) were used for the generation of the cell pattern. PnV Litho was carried out as previously explained, using IO-3 and GelMa as gels for the formation of the structures. PDMS slabs were removed and the wells were loaded with 1 mL of blocking solution (BSA 5 %, milk powder 0.1 % and casein 1 % in PBS) for 2 h. Then, hHF-MSCs were detached from the flasks and were resuspended in serum free medium (SFM1) at a concentration of 10⁵ cells mL⁻¹. 750 µL of the cell suspension were loaded to the pattern of fibronectin surrounded by ionogel, and were left inside the incubator on constant oscillation in a rocker (Vari-Mix steep angle rocker, Thermo Fisher) for 60 min (n = 3 per experimental condition). Afterwards, the medium was retrieved and the wells were rinsed three times with PBS to wash out any non-attached cell.

### 1.4 - FGF-2 presentation on co-patterned GelMa gels and HeLa cells.

To combine the GelMa structures and cells, Printing and Vacuum Lithography (PnV Litho) was carried out. Briefly, GelMa was heated at 40 °C for 1 h while carrying out PnV litho. To evaluate the presentation of FGF-2 on growth factor-loaded GelMa, the gel was mixed with FGF-2 (100 µg mL⁻¹) in a 1:10. PDMS slabs channel-like structures (1000 x 5000 x 13 µm, width x length x height) with pillars inside (100 µm diameter) were used for the generation of the cell pattern. PnVlitho protocol was followed as explained in Section 2.3. In this case, the pillars inside of the channels were wetted with a solution of fibronectin 100 µg mL⁻¹ for 30 min. Polymerization was carried out by exposing the samples to UV light for 20 cycles of 5 s, every 30 s. As controls, GelMa was loaded with H₂O in the same proportion. Three samples were done per experimental condition. PDMS slabs attached to the glass covers were left overnight for solvent evaporation at room temperature.

All wells were blocked with 1 mL of blocking solution (BSA 5 %, milk powder 0.1 % and casein 1 % in PBS). Subsequently, wells were loaded with 750 µL of Hela suspension 2 10⁵ cells mL⁻¹ in SFM2 and left inside the incubator for 1 h in static. Finally, the medium was retrieved from all wells; the wells were rinsed three times with PBS and replenished with 750 µL of fresh SFM2. All the samples were left inside the incubator for 24 h. At the end of the experiment, the cells were photographed and counted.

### 1.5 - VEGF secretion and FGF-2 stimulation

To detect the secretion of VEGF and to present FGF-2, multicomponent patterns of three different materials, including GelMa, fibronectin and microbeads were tested. The patterning of GelMa doped with FGF-2 and microbeads functionalized with anti-VEGF antibodies was carried out through PnV litho. For the microbead preparation, the stock microbead suspensions were centrifuged at 10000 rpm for 15 min. Afterwards, the microbeads were resuspended with 100 µL of VEGF-Ab (5 µg mL⁻¹) and were left incubating at room temperature for 60 min. Then, the suspensions were centrifuged at the same conditions. Finally, the suspensions were resuspended in distilled water for a final concentration of 8 10¹⁰ microbeads mL⁻¹. GelMa gels were either mixed with FGF-2 (100 µg mL⁻¹) or with H₂O in a 1:10 proportion.

PDMS slabs wave channel-like structure were used (two parallel wave-like channels of 400 µm width × 8200 µm length × 33 µm height separated 100 µm between channels). The separation between the channels was wetted with a solution of fibronectin 100 µg mL⁻¹ for 30 min. Afterwards, PDMS slabs were rinsed with distilled water, dried with compressed air and attached to the glass cover previously functionalized. The PnVlitho as described in Section 2.3., loading GelMa in one of the channels and the microbeads suspension in the other. PDMS slabs attached to the glass covers were left overnight for solvent evaporation at room temperature.

After solvent evaporation and PDMS removal, the pattern generated, were incubated with 1 mL of blocking solution (BSA 5 %, milk powder 0.1 % and casein 1 % in PBS). Afterwards, cells were detached and resuspended in SFM2 for a concentration of 2 10⁵ cells mL⁻¹. 750 µL of the suspension of cells were loaded on top of the pattern and were left secreting for 48 h in the incubator. Afterwards, the samples wells were rinsed three times and cells were fixed with paraformaldehyde 4 % for 10 min. Wells were incubated with primary Ab and secondary Ab and brightfield and fluorescence microscopy images were taken for analysis of the results.

### 2. Results

### 2.1. Generation of gel microstructures through vacuum lithography

In order to generate microstructures using vacuum-driven soft lithography, three different ionogels (IO-1, IO-2 and IO-3) and GelMa were evaluated. lonogels are polymeric hybrid materials, which has risen interest for different application such as sensors, electronics and biomedicine. The ionogels studied consisted on NIPAAm, the crosslinker mBAAm, the photoinitiator DMPA and the ionic liquid (IL), which in this case were EMIES, DCA and ChAc. This ILs has a variable nature, conferring the ionogel with different physicochemical properties, like ChAc that made the ionogel biocompatible and hygroscopic. In the other hand, GelMa is a gelatin-based biomaterial widely used for various biomedical applications such as tissue engineering, due to their suitable biological properties and tunable physical characteristics.

For that, different polymerization cycles were studied, in order to determine the desirable conditions for gel polymerization. Homogeneity of the gels, as well as the total height obtained were evaluated. Different UV light cycles were studied for the polymerization of ionogels (5, 10, 20, 30, 50 and 70 cycles) and GelMa (10 and 20 cycles). After removing the PDMS stamps, optical profilometry was used to obtain the height of the microstructures generated with the different polymerization cycles (Figure 1). For the IO-1 the height vary between 9 µm ± 2 µm (5 cycles) to 14 µm ± 2 µm (30 cycles) (18 measured from n=3 samples). In the case of IO-2, the range of height obtained was 8 µm ± 1 µm (10 cycles) and 15 µ ± 3 µm (30 cycles) (18 measured from n=3 samples). The IO-3 microstructure height were between 9 µm ± 0.3 µm (5 cycles) to 13 µm ± 2 µm (70 cycles) (18 measured from n=3 samples).

In the case of the ionogel structures, the height obtained for each one depended mostly on the number of UV cycles they were exposed to and not the composition of the gel, Figure 1. In particular, IO-2 structures presented highest height variance between 10 and 30 cycles, whereas IO-3 presented the lowest. It should be noted that, in all cases, the gel structures obtained presented only a fraction of the maximum height available by the channel (33 µm), resulting in an average percentage of the channel height of 42 % ± 6, 45 % ± 9 and 39 % ± 6 for IO-1, IO-2 and IO-3, respectively.

The height of the GelMa microstructures obtained were quite similar between each other, presenting 3.7 µm ± 0.1 µm (10 cycles) to 3.6 µm ± 0.1 µm (20 cycles) (18 measured from n=3 samples). GelMa structures presented the lowest height out of all the gels tested, as the mean heights obtained represented only 11 % ± 0.3.

### 2.2- Co-patterning of IO-3 and hHF-MSCs

Combined patterns of wavy IO-3 structures with a fibronectin path between them were generated by PnV Litho, and the subsequent patterning of cells to produce a wavy channel of cells, surrounded by ionogel. These patterns were then incubated with a hHF-MSCs suspension, where the cells adhered to the fibronectin path between the two gel structures. The result was a good pattern of fibronectin surrounded by the IO-3 channels on the glass substrates, as revealed by brightfield microscopy. As seen in Figure 2, brightfield microscopy showed a good definition of the IO-3 structures and that cells adhered specifically on the protein channel previously printed. The cell pattern obtained was stained with DAPI and phalloidin (Figure 2 B) or with the Live/Dead viability kit (Figure 2 C) to check the state of the cells in that surrounding. The cell displayed a good morphology, as they are spread between the two microstructures. However, cells seem to die over the time as some of the cells nucleus in the live/dead assay are red, indicating the cell death. This can be attributed to IO-3 being biocompatible but not cytocompatible,

Afterwards, patterns of fibronectin dots (100 µm diameter) surrounded by either IO-3 or GelMa were fabricated. hHF-MSCs were then incubated on the patterns, in order to generate small islands of hHF-MSCs surrounded by one of the two gels. As seen in Figure 3, cells did not properly attached to the fibronectin dots in the samples containing IO-3, indicating that the architecture of the adhesion zones is a fundamental factor for cell binding. By contrast, cells perfectly adhere to the fibronectin dots in the GelMa patterns, producing dozens of small hHF-MSCs islands surrounded by the gel. These demonstrates not only the capability to form combined patterns of adhesion proteins and gels, and therefore cells and gels, but how the chemical composition of the three-dimensional structures affect cell adhesion.

### 2.3 -Presentation of FGF-2 co-patterns of HeLa islands surrounded by GelMa microstructures

The co-patterned of GelMa doped with FGF-2 and HeLa were used to evaluate the ability of the microstructure to present soluble factors to the cells. The GelMa solution loaded were either combined with H2O (FGF-) or doped with FGF-2 (100 µg/mL, FGF+) at a 1:10 proportion. HeLa cells were patterned in small islands (100 µm) surrounded by GelMa, and were incubated for 48 h in intimate contact with the GelMa microstructures. Finally, the cells were photographed through brightfield microscopy.

Our results showcase the effect of doping GelMa with FGF-2 (Figure 4). As the cells were maintained in serum-free conditions, a prevalence of cell death over the 48 h was expected. In the case of the cells patterned with bare GelMa (FGF -), only a third of the original cells remained in the patterns (33 % ± 9) after 48h. In contrast, more than a half of the cells patterned in GelMa doped with FGF-2 (FGF +) remained after 48 h (55.5 % ± 0.9), confirming the positive effect presented by the GELMA on cell survival. Moreover, differences in the morphology of the cells can be seen at 48 h comparing the two conditions. Due to the presence of FGF, the cells shows a stretched morphology, while in its absence, they present a round morphology, an indicator of cell death or discomfort. It should be noted that, throughout the 48 h, GelMa structures started to decrease in height, which can be attributed to a digestion of the collagen-derived material by enzymes secreted by the cells.

### 2.4 - Combined FGF-2 presentation and secreted VEGF detection on multicomponent patterns of HeLa cells, GelMa microstructures and microbeads

HeLa cells were patterned in a small path (100 µm), with a microbeads pattern at one side (functionalized with anti-VEGF Antibody) and a pattern of GelMa either combined with H₂O (FGF-) or doped with FGF-2 (100 µg/mL, FGF+). Cells were maintained secreting VEGF for 48 h inside an incubator. In all samples where HeLa patterns were present, higher fluorescence intensity could be observed in the microbeads patterns when compared to the control samples without cells. Our results showcased the effect of doping GelMa with FGF-2 in VEGF secretion, Figure 4. Cells patterned with GelMa doped with FGF-2 doubled the secretion of the growth factor. These results prove the effect of the FGF-2 presence in the GelMa has on the patterned cells on the secretion of other growth factors such as VEGF.

## Claims

1. A support suitable for culturing cells **characterized in that** it shows patterned regions, said patterned regions being defined by:
- a complementary pattern of a uniformly distributed gel attached to the support, wherein the gel comprises at least one first functionalizing molecule, or
- a complementary pattern comprising two or more complementary patterned regions, wherein at least one complementary pattern region is defined by an uniformly distributed gel attached to the support and at least one complementary pattern region is defined by uniformly distributed microbeads attached to the support, wherein the gel comprise at least one first functionalizing molecule and/or the microbeads are modified with at least one second functionalizing molecule,
wherein the patterned regions in the support show increased adhesive capacity to cells with respect to the complementary pattern to which the gel or the gel and microbeads are attached.

2. The support according to any of claim 1 wherein the regions in the support onto which the gel or gel and microbeads are attached are further coated with a compound or composition which substantially reduces cell adhesion.

3. The support according to claim 1 or 2 wherein the patterned regions are created using vacuum-driven soft lithography.

4. The support according to any one of claims 1 to 3 wherein the gel is a hydrogel or an ionogel.

5. The support according to claim 4 wherein the hydrogel polymer is gelatin, preferably gelatin methacrylate.

6. The support according to any of claims 1 to 5 wherein the patterned regions in the support are coated with a compound or composition that promotes cell adhesion.

7. The support according to claim 6 wherein the compound that promotes cell adhesion is an extracellular matrix molecule, preferably fibronectin.

8. The support according to any of claims 1 to 7 wherein the patterned regions have a geometrical form, preferably a circular form with a diameter of between 10 µm to 200 µm.

9. The support according to any of claims 1 to 8 wherein the support further contains viable cells which are attached to the support or to molecules within the support which promote cell adhesion.

10. A method for producing the support according to any of claims 1 to 9 selecting from:
(a) a method comprising the steps of:
(i) Providing a support;
(ii) Contacting the support with a stamp, said stamp having protrusions which define the desired pattern in the support, wherein the contact of the support with the stamp forms bottomless channels between the support and the stamp,
(iii) contacting the support along the bottomless channels with a gel solution and allowing the gel solution to distribute uniformly in those regions in the support which are not protected by the protrusions of the stamp, and
(iv) polymerizing the gel in order for the gel to adhere to the support as to obtain a uniform attachment onto those regions in the support which are not protected by the protrusions of the stamp obtaining a pattern defined by the gel which is complementary to the desired pattern,
wherein the gel solution comprises at least one first functionalizing molecule or at least one first functionalizing molecule is added to the gel after the gel is polymerized;
or
(b) a method comprising the steps of:
(i) Providing a support;
(ii) Contacting the support with a stamp, said stamp having protrusions which define the desired pattern in the support, wherein the contact of the support with the stamp forms bottomless channels between the support and the stamp,
(iii) contacting the support along the bottomless channels with a gel solution and a microbead suspension wherein the microbeads are modified with at least one second functionalizing molecule and allowing the microbeads to adhere to the support as to obtain a uniform deposition in and attachment of the microbeads onto those regions in the support which are not protected by the protrusions of the stamp nor occupied by the gel solution, and
(iv) and polymerizing the gel in order for the gel to adhere to the support as to obtain a uniform attachment onto those regions in the support which are not protected by the protrusions of the stamp nor occupied by the microbeads, obtaining a pattern defined by the gel and microbeads which is complementary to the desired pattern,
wherein the gel comprises at least one first functionalizing molecule and/or the microbeads are modified with at least one second functionalizing molecule and wherein the gel solution comprises at least one first functionalizing molecule or at least one first functionalizing molecule is added to the gel after the gel is polymerized.

11. The method according to claim 10 wherein the regions in the support onto which the microbeads attaches in step (iii) of method (b) and onto which the gel attaches in step (iv) of method (a) and (b) are further coated with a compound or composition which substantially reduces cell adhesion.

12. The method according to claim 10 or 11 wherein in step (iii) of method (a) and (b) the chamber formed between the stamp and the support is provided with at least one operable inlet and optionally at least one operable outlet.

13. The method according to any of claims 10 to 12 wherein in step (iii) and step (iv) of method (a) and (b) the gel solution or the gel solution and the microbead suspension further comprises a compound or composition which substantially reduces cell adhesion or a compound of composition of interest.

14. A method for determining the effect of a molecule on a cell population which comprises the steps of:
(i) Providing a support according to any of the claims 1 to 9 wherein said support contains the cell population wherein the cells of the population are attached to the support and wherein the molecule, the effect of which on the cell population is to be determined is the at least one first functionalizing molecule and/or the at least one second functionalizing molecule;
(ii) Maintaining the support under adequate conditions to allow the at least one first functionalizing molecule and/or the at least one second functionalizing molecule to exert its effect on the cells of the cell population; and
(iii) Detecting the response of the cells within the cell population to said first functionalizing molecule and/or the at least one second functionalizing molecule,
wherein the detection of a response of the cells to the at least one first functionalizing molecule and/or the at least one second functionalizing molecule is indicative that the at least one first functionalizing molecule and/or the at least one second functionalizing molecule exerts an effect on the cells of the cell population.

15. A method for determining if a cell population secretes to the medium a substance of interest which comprises the steps of:
(i) Providing a support according to any of the claims 1 to 9 wherein said support contains a cell population wherein the cells of the population are attached to the support and wherein the at least one first functionalizing molecule and/or the at least one second functionalizing molecule comprises a molecule which is capable of interacting with the substance of interest, said interaction resulting in a detectable signal;
(ii) Culturing the cells in the adequate conditions to allow binding of the substances of interest secreted to the medium to the at least one first functionalizing molecule and/or the at least one second functionalizing molecule; and
(iii) Detecting the binding of the substance of interest to the at least one first functionalizing molecule and/or the at least one second functionalizing molecule by detecting the signal resulting from said interaction.
